# FASCICULE DE BREVET EUROPEEN

(11) **EP 1 537 096 B1**
(45) Date de publication et mention de la délivrance du brevet: **24.09.2008**
(21) Numéro de dépôt: 03758282.2
(22) Date de dépôt: 27.08.2003
(51) Int. Cl.: C07D 319/06, C07D 405/12, A61K 31/335, A61P 25/08, C07D 413/06

(54) **DERIVES DE DIOXANE-2-ALKYLCARBAMATES, LEUR PREPARATION ET LEUR APPLICATION EN THERAPEUTIQUE**
DIOXAN-2-ALKYLKARBAMATEN DERIVATEN, DEREN HERSTELLUNG UND DEREN THERAPEUTISCHEN VERWENDUNG
DERIVATIVES OF DIOXANE-2-ALKYL CARBAMATES, PREPARATION METHOD THEREOF AND APPLICATION OF SAME IN THERAPEUTICS

(30) Priorité: 29.08.2002 FR 0210707
(43) Date de publication de la demande: 08.06.2005
(60) Demande divisionnaire: 07013954.8
(73) Titulaire: Sanofi-Aventis, 75013 Paris (FR)
(72) Inventeur: ABOUABDELLAH, Ahmed, F-94320 Thiais (FR); BAS, Michèle, F-34570 Pignan (FR); DARGAZANLI, Gihad, F-94230 Cachan (FR); HOORNAERT, Christian, F-92160 Antony (FR); LI, Adrien, Tak, F-92260 Fontenay aux Roses (FR); MEDAISKO, Florence, F-94100 Saint Maur des Fosses (FR)
(74) Mandataire: Monain, Patrice
(86) Numéro de dépôt international: PCT/FR2003/002590
(87) Numéro de publication internationale: WO 2004/020430

(56) Documents cités:
- EP-A- 0 461 958
- WO-A-97/20836

## Description

L'invention a pour objet des dérivés de 1,3-dioxan-2-ylalkylcarbamates, leur préparation et leur application en thérapeutique.

Les composés de l'invention répondent à la formule générale (I) 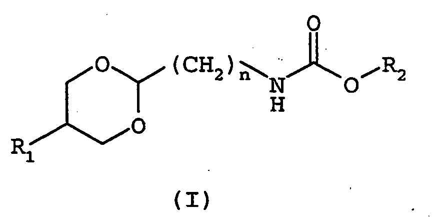 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ;
R₂ représente
soit un groupe de formule générale CHR₃CONHR₄ dans laquelle
R₃ représente un atome d'hydrogène ou un groupe méthyle et
R₄ représente un atome d'hydrogène ou un groupe C₁₋₃-alkyle, C₃₋₅-cycloalkyle ou (pyridin-4-yl)méthyle,
soit un groupe 2,2,2-trifluoroéthyle,
soit un groupe (imidazol-2-yl)méthyle,
soit un groupe (benzimidazol-2-yl)méthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy ; et
n représente un nombre allant de 1 à 3.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces énantiomères, diastéréoisomères et stéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Des composés analogues à ceux de l'invention, pour lesquels R₂ représente un groupe C₁₋₄-alkyle linéaire ou ramifié, ont été décrits en tant que anticonvulsivants dans les documents EP0461958 et FR2714056.

Dans le cadre de l'invention, on entend par :
- C_{t-z} où t et z peuvent prendre les valeurs de 1 à 6, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple, un groupe C₁₋₃-alkyle représente une chaîne carbonée de 1 à 3 atomes de carbone, linéaire ou ramifiée, plus particulièrement un méthyle, éthyle, propyle, isopropyle ;
- cycloalkyle, un groupe alkyle cyclique, par exemple, un groupe C₃₋₅-cycloalkyle représente une chaîne carbonée cyclique de 3 à 5 atomes de carbone, plus particulièrement un cyclopropyle, cyclobutyle, cyclopentyle ;
- alcoxy, un groupe alkyloxy à chaîne aliphatique saturée, linéaire ou ramifiée ;
- atome d'halogène, un fluor, un chlore, un brome ou un iode.

Parmi les composés de formule générale (I), on peut citer des composés préférés qui se définissent comme suit :
R₁ représente un groupe naphtalènyle, éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ; et/ou
R₂ représente
soit un groupe de formule générale CHR₃CONHR₄ dans laquelle
R₃ représente un atome d'hydrogène et
R₄ représente un atome d'hydrogène ou un groupe C₁₋₃-alkyle, de préférence un méthyle ou un éthyle, ou (pyridin-4-yl)méthyle,
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy;
et/ou
n représente 2 ou 3.

Les composés pour lesquels à la fois R₁, R₂ et n sont tels que définis ci-dessus sont particulièrement préférés.

A titre d'exemple de composés préférés, on peut citer les composés suivants :
*trans*-3-[5-(naphtalèn-1-yl)-1,3-dioxan-2-yl]propylcarbamate de 2-amino-2-oxoéthyle.
*trans*-2-[5-(naphtalèn-1-yl)-1,3-dioxan-2-yl]éthylcarbamate de 2-(méthylamino)-2-oxoéthyle.
*trans*-3-[5-(naphtalèn-1-yl)-1,3-dioxan-2-yl]propylcarbamate de 2-(méthylamino)-2-oxoéthyle.
*trans*-2-[5-(naphtalèn-1-yl)-1,3-dioxan-2-yl]éthylcarbamate de 2,2,2-trifluoroéthyle.
*trans*-3-[5-(naphtalèn-1-yl)-1,3-dioxan-2-yl]propylcarbamate de 2,2,2-trifluoroéthyle.
*trans*-2-[5-(naphtalèn-1-yl)-1,3-dioxan-2-yl]éthylcarbamate de phényle.
*trans*-3-[5-(naphtalèn-1-yl)-1,3-dioxan-2-yl]propylcarbamate de phényle.
*trans*-3-[5-(4-chloronaphtalèn-1-yl)-1,3-dioxan-2-yl]propylcarbamate de 2-amino-2-oxoéthyle.
*trans*-3-[5-(4-chloronaphtalèn-1-yl)-1,3-dioxan-2-yl]propylcarbamate de 2-(méthylamino)-2-oxoéthyle*.*
*trans*-3-[5-(6-chloronaphtalèn-1-yl)-1,3-dioxan-2-yl]propylcarbamate de 2-(méthylamino)-2-oxoéthyle.
*trans*-3-[5-(6-méthoxynaphtalèn-1-yl)-1,3-dioxan-2-yl]propylcarbamate de 2-amino-2-oxoéthyle.
*cis*-3-[5-(6-méthoxynaphtalèn-1-yl)-1,3-dioxan-2-yl]propylcarbamate de 2-amino-2-oxoéthyle.
*trans*-2-[5-(6-méthoxynaphtalèn-1-yl)-1,3-dioxan-2-yl]éthylcarbamate de 2-(méthylamino)-2-oxoéthyle.
*trans*-2-[5-(6-méthoxynaphtalèn-1-yl)-1,3-dioxan-2-yl]éthylcarbamate de 4-chlorophényle.
*trans*-2-[5-(6-méthoxynaphtalèn-1-yl)-1,3-dioxan-2-yl]éthylcarbamate de 2,2,2-trifluoroéthyle.
*trans*-3-[5-(6-méthoxynaphtalèn-1-yl)-1,3-dioxan-2-yl]propylcarbamate de 2-(méthylamino)-2-oxoéthyle.
trans-3-[5-(6-méthoxynaphtalèn-1-yl)-1,3-dioxan-2-yl]propylcarbamate de 2-(éthylamino)-2-oxoéthyle.
*trans*-3-[5-(6-méthoxynaphtalèn-1-yl)-1,3-dioxan-2-yl]propylcarbamate de 2-[(pyridin-4-yl)méthylamino]-2-oxoéthyle.
*trans*-3-[5-(6-méthoxynaphtalèn-1-yl)-1,3-dioxan-2-yl]propylcarbamate de 2,2,2-trifluoroéthyle.
*trans*-3-[5-(6-méthoxynaphtalèn-1-yl)-1,3-dioxan-2-yl]propylcarbamate de phényle.
*trans*-3-[5-(6-cyclopropylméthoxynaphtalèn-1-yl)-1,3-dioxan-2-yl]propylcarbamate de 2-amino-2-oxoéthyle.
*trans*-3-[5-(6-cyclopropylméthoxynaphtalèn-1-yl)-1,3-dioxan-2-yl]propylcarbamate de 4-chlorophényle.
*trans*-3-[5-(6-cyclopropylméthoxynaphtalèn-1-yl)-1,3-dioxan-2-yl]propylcarbamate de 2,2,2-trifluoroéthyle.
*trans*-3-[5-(6-phénylméthoxynaphtalèn-1-yl)-1,3-dioxan-2-yl]propylcarbamate de 2-amino-2-oxoéthyle.
*trans*-3-[5-(6-hydroxynaphtalèn-1-yl)-1,3-dioxan-2-yl]propylcarbamate de 2-amino-2-oxoéthyle.
*trans*-3-[5-(6-hydroxynaphtalèn-1-yl)-1,3-dioxan-2-yl]propylcarbamate de 2-(méthylamino)-2-oxoéthyle.
*trans*-3-[5-(7-méthoxynaphtalèn-1-yl)-1,3-dioxan-2-. yl]propylcarbamate de 2-amino-2-oxoéthyle.
*trans*-3-[5-(7-méthoxynaphtalèn-1-yl)-1,3-dioxan-2-yl]propylcarbamate de 2-(méthylamino)-2-oxoéthyle.
*trans*-3-[5-(7-méthoxynaphtalèn-1-yl)-1,3-dioxan-2-yl]propylcarbamate de 2,2,2-trifluoroéthyle.
*trans*-3-[5-(7-méthoxynaphtalèn-1-yl)-1,3-dioxan-2-yl]propylcarbamate de phényle.
*trans*-3-[5-(naphtalèn-2-yl)-1,3-dioxan-2-yl]propylcarbamate de phényle.
*trans*-3-[5-(naphtalèn-2-yl)-1,3-dioxan-2-yl]propylcarbamate de 2-(méthylamino)-2-oxoéthyle.
*trans*-3-[5-(naphtalèn-2-yl)-1,3-dioxan-2-yl]propylcarbamate de 2,2,2-trifluoroéthyle.

Les composés de l'invention peuvent être préparés selon différentes méthodes, illustrées par les schémas qui suivent.

Ainsi une méthode de préparation (schéma 1) consiste à faire réagir une amine de formule générale (II), dans laquelle R₁ et n sont tels que définis dans la formule générale (I), avec un carbonate de formule générale (III), dans laquelle U représente un atome d'hydrogène ou un groupement nitro et R₂ est tel que défini dans la formule générale (I), dans un solvant tel que le toluène ou le dichloroéthane, à une température comprise entre 0 et 80°C.

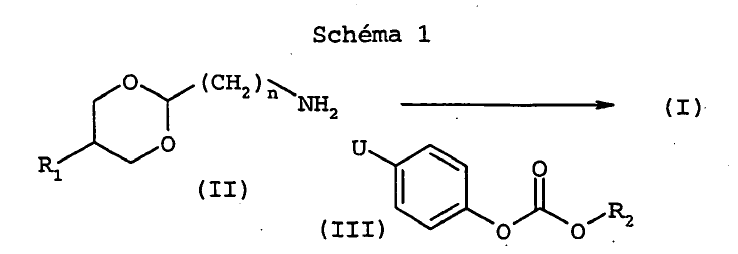

Les carbonates de formule générale (III) peuvent être préparés selon toute méthode décrite dans la littérature, par exemple par réaction d'un alcool de formule générale HOR₂ avec le chloroformiate de phényle ou de 4-nitrophényle, en présence d'une base telle que la triéthylamine ou la diisopropyléthylamine.

Les composés de formule générale (I) pour lesquels R₂ représente plus particulièrement un groupement phényle éventuellement substitué (Ar) peuvent être préparés en faisant réagir une amine de formule générale (II), telle que définie ci-dessus, avec un chloroformiate d'aryle de formule générale (IIIa) dans un solvant tel que le dichlorométhane ou le dichloroéthane en présence d'une base telle que la triéthylamine ou la diisopropyléthylamine, à une température comprise entre 0°C et la température de reflux du solvant.

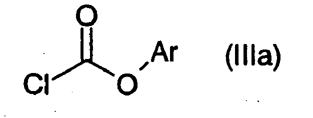

Selon le schéma 2, les composés de formule générale (I) pour lesquels R₂ représente plus particulièrement un groupement de formule générale CHR₃CONHR₄ peuvent être préparés en faisant réagir une amine de formule générale (II), telle que définie ci-dessus, avec du dioxyde de carbone en présence d'une base telle que le carbonate de césium et d'un agent de transfert de phase tel que l'iodure de tétra-n-butylammonium, dans un solvant tel que le N,N-diméthylformamide ou la N-méthylpyrrolidone, puis avec un haloacétamide de formule générale (IV) dans laquelle V représente un atome de chlore, brome ou iode et R₃ et R₄ sont tels que définis dans la formule générale (I).

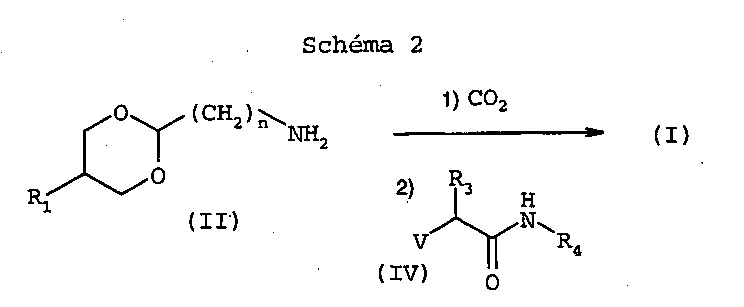

Une variante (schéma 3) pour obtenir les composés de formule générale (I) dans laquelle R₂ représente plus particulièrement un groupement de formule générale CHR₃CONHR₄ consiste à faire réagir une amine de formule générale (II), telle que définie ci-dessus, avec un carbonate de formule générale (IIIb) dans laquelle U représente un atome d'hydrogène ou un groupe nitro, R₃ est tel que défini dans la formule générale (I) et R₅ représente un groupe méthyle ou éthyle. Le carbamate-ester de formule générale (Ia) ainsi obtenu est ensuite transformé en composé de formule générale (I), soit par aminolyse directe au moyen d'une amine de formule générale R₄NH₂ dans laquelle R₄ est tel que défini dans la formule générale (I), soit par hydrolyse en acide de formule générale (Ia), dans laquelle R₅ représente un atome d'hydrogène, suivie d'un couplage avec une amine de formule générale R₄NH₂ dans laquelle R₄ est tel que défini dans la formule générale (I). La réaction d'aminolyse peut être réalisée dans un solvant tel que le méthanol ou un mélange de solvants tels que le méthanol et le tétrahydrofurane. La réaction de couplage peut être réalisée selon toute méthode connue de la littérature, par exemple avec utilisation de chloroformiate d'isobutyle, en présence d'une base telle la diisopropyléthylamine.

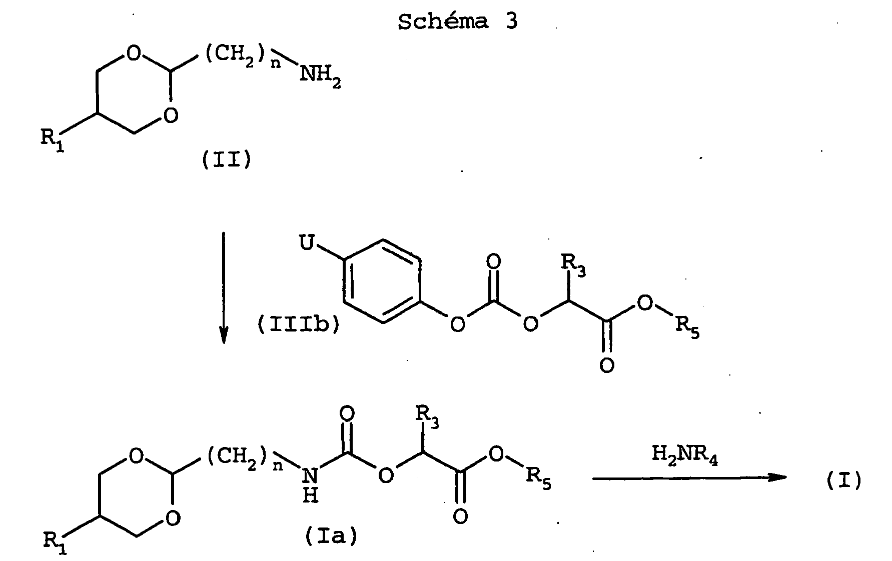

Les carbonates de formule générale (IIIb) peuvent être préparés de manière analogue aux carbonates de formule (III).

Une autre variante (schéma 4) pour obtenir les composés de formule générale (I) dans laquelle R₂ représente plus particulièrement un groupement de formule générale CHR₃CONHR₄, consiste à faire réagir un dérivé de formule générale (IIa) dans laquelle Z représente un groupement hydroxyle, mésylate, tosylate, ou un atome de chlore, de brome ou d'iode, R₁ et n sont tels que définis dans la formule générale (I), avec une oxazolidine-dione de structure générale (V) dans laquelle R₃ est tel que défini dans la formule générale (I), pour fournir le dérivé oxazolidine-dione de structure générale (VI). Dans le cas où Z représente un groupement hydroxyle, la réaction peut être conduite suivant les conditions de Mitsunobu (Synthesis, 1981, 1-28), par exemple, par action d'azodicarboxylate de diéthyle ou de diisopropyle en présence de triphénylphosphine. Dans le cas où Z représente un atome de chlore, de brome, ou d'iode, ou un groupement mésylate ou tosylate, la réaction peut être conduite en présence d'une base telle que la 1,1,3,3-tétraméthylguanidine, l'hydrure de sodium ou le tert-butoxyde de sodium dans un solvant tel que le tétrahydrofurane, l'acétonitrile ou le diméthylformamide à une température comprise entre 0°C et la température de reflux du solvant. Le dérivé oxazolidine-dione de formule générale (VI) ainsi obtenu est ensuite transformé en composé de formule générale (I), par aminolyse au moyen d'une amine de formule générale R₄NH₂ où R₄ est tel que défini dans la formule générale (I).

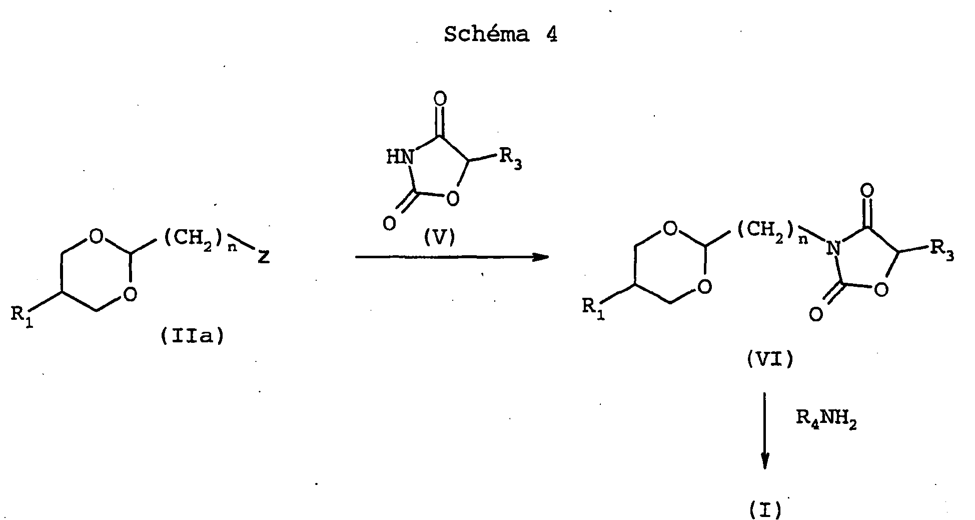

Les amines de formule générale (II) peuvent être préparées selon les méthodes de préparation décrites dans les demandes de brevets EP0461958, WO97/20836 WO98/55474 éventuellement adaptées suivant des techniques connues de l'homme du métier.

Les composés de formules générales (IIa), (IIIa), (IV) et (V), ainsi que les amines R₄NH₂, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les composés de formule générale (Ia), dans laquelle R₁, R₃ et n sont tels que définis dans la formule générale (I) et R₅ représente un atome d'hydrogène, un groupe méthyle ou éthyle, et les composés de formule générale (VI), dans laquelle R₁, R₃ et n sont tels que définis dans la formule générale (I), sont nouveaux et font également partie de l'invention. Ils sont utiles en tant qu'intermédiaires de synthèse pour la préparation des composés de formule générale (I).

Les exemples qui vont suivre illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
Les numéros indiqués entre parenthèses dans les titres des exemples correspondent à ceux de la 1ère colonne du tableau ci-après.

### Exemple 1 (Composé N°61).

*trans*-3-[5-(6-méthoxynaphtalèn-1-yl)-1,3-dioxan-2-yl]propylcarbamate de 2-(cyclopropylamino)-2-oxoéthyle.

### 1.1. [(phénoxycarbonyl)oxy]acétate d'éthyle.

A une solution de 10 g (96,15 mmoles) de glycolate d'éthyle et 27 ml (192,3 mmoles) de triéthylamine dans 20 ml de toluène on ajoute, goutte à goutte, à température ambiante, 13,5 ml (105,6 mmoles) de chloroformiate de phényle et on agite le mélange à température ambiante pendant 2 h. On sépare le sel formé et on concentre le filtrat sous pression réduite.
On obtient 20 g de produit huileux qu'on utilise tel quel dans l'étape suivante.

### 1.2. trans-[[[[3-[5-(6-méthoxynaphtalèn-1-yl)-1,3-dioxan-2-yl]propyl]amino]carbonyl]oxy]acétate d'éthyle.

On chauffe à 50°C pendant 12 h une solution de 10 g (33 mmoles) de trans-3-[5-(6-méthoxynaphtalèn-1-yl)-1,3-dioxan-2-yl]propanamine et 8,9 g (39,8 mmoles) de [(phénoxycarbonyl)oxy]acétate d'éthyle, obtenu à l'étape 1.1, dans 500 ml de toluène. On laisse revenir à température ambiante, on sépare l'insoluble par filtration et on concentre le filtrat sous pression réduite. On reprend le résidu par du dichlorométhane et de l'eau, on sépare la phase aqueuse et on l'extrait trois fois avec du dichlorométhane, on lave les phases organiques réunies avec une solution aqueuse saturée en chlorure de sodium et on les sèche sur sulfate de sodium. Après évaporation du solvant on purifie le résidu par chromatographie sur gel de silice en éluant avec un mélange 20/80 d'acétate d'éthyle et de cyclohexane.
On obtient finalement 7 g de produit pur sous forme d'huile qui cristallise.
Point de fusion : 74-76°C.

### 1.3. Acide trans-[[[[3-[5-(6-méthoxynaphtalèn-1-yl)-1,3-dioxan-2-yl]propyl]amino]carbonyl]oxy]acétique.

A une solution de 4 g (9,27 mmoles) de trans-[[[[3-[5-(6-méthoxynaphtalèn-1-yl)-1,3-dioxan-2-yl]propyl]amino]-carbonyl]oxy]acétate d'éthyle, obtenu à l'étape 1.2, dans 40 ml de diméthoxyéthane on ajoute, goutte à goutte, 40 ml d'une solution aqueuse d'hydroxyde de sodium 1N, et on agite le mélange à température ambiante pendant 2 h. On concentre sous pression réduite, on dissout le résidu dans un minimum d'eau, on ajoute de l'acide chlorhydrique 1N jusqu'à l'obtention d'un pH égal à 4, on extrait la phase aqueuse trois fois avec du dichlorométhane, on sépare la phase organique, on la sèche sur sulfate de sodium, on concentre sous pression réduite.
On obtient 3 g d'acide.
Point de fusion : 114-116°C.

### 1.4. trans-3-[5-(6-méthoxynaphtalèn-1-yl)-1,3-dioxan-2-yl]propylcarbamate de 2-(cyclopropylamino)-2-oxoéthyle.

A une solution de 0,5 g (1,24 mmoles) d'acide trans-[[[[3-[5-(6-méthoxynaphtalèn-1-yl)-1,3-dioxan-2-yl]propyl]amino]carbonyl]oxy]acétique, obtenu à l'étape 1.3, et 0,65 ml (3,70 mmoles) de N,N-diisopropyléthylamine dans 10 ml de tétrahydrofurane, refroidie à environ -20°C, on ajoute, goutte à goutte, sous atmosphère inerte, une solution de 0,169 g (1,24 mmoles) de chloroformiate d'isobutyle dans 5 ml de tétrahydrofurane tout en maintenant la température du milieu réactionnel inférieur à -15°C. On maintient l'agitation à cette température pendant 1 h, puis on ajoute lentement une solution de 0,078 g (1,36 mmoles) de cyclopropylamine dans 5 ml de tétrahydrofurane, et on poursuit l'agitation à -15°C pendant 1 h, puis à 20°C pendant 10 h. On concentre sous pression réduite, on reprend le résidu avec de l'acétate d'éthyle et de l'eau, on sépare la phase organique, on la lave avec une solution aqueuse saturée en chlorure de sodium, on la sèche sur sulfate de sodium, on concentre sous pression réduite et on recristallise le solide dans l'éthanol.
On obtient finalement 0,258 g de produit pur.
Point de fusion : 176°C.
RMN ¹H : (CDCl₃) δ (ppm) 8, 10 (d, 1H); 7,65 (d, 1H) ; 7,35 (dd, 1H); 7,25 (d, 1H); 7,15 (dd, 1H); 7,05 (d, 1H); 6,20 (m large 1H); 5,05 (m large, 1H); 4,70 (t, 1H); 4,55 (s, 2H); 4,35 (m, 2H); 3,95-3,90 (m, 6H); 3,30 (m, 2H); 2,75 (m, 1H); 1,75 (m, 4H); 0,80 (m, 2H); 0,55 (m, 2H).

### Exemple 2 (Composé N°49).

*trans-*3-[5-(6-méthoxynaphtalèn-1-yl)-1,3-dioxan-2-yl]propylcarbamate de 2-amino-2-oxoéthyle.

Dans un ballon tricol de 1 l placé sous atmosphère inerte on introduit 20 g (66 mmoles) de trans-3-[5-(6-méthoxynaphtalèn-1-yl)-1,3-dioxan-2-yl]propanamine, 64 g (198 mmoles) de carbonate de césium et 73,14 g (198 mmoles) d'iodure de tétra-n-butylammonium en suspension dans 400 ml de *N,N*-diméthylformamide. On fait passer un courant de dioxyde de carbone dans la suspension, sous forte agitation, pendant 2 h. On ajoute ensuite, goutte à goutte, 18,5 g (198 mmoles) de chloroacétamide en solution dans 70 ml de *N,N*-diméthylformamide, et on maintient le courant de dioxyde de carbone pendant 5 h, et on poursuit l'agitation à température ambiante pendant une nuit. On sépare les sels par filtration, on concentre le filtrat sous pression réduite, on reprend le résidu avec de l'acétate d'éthyle et de l'eau, on sépare la phase organique, on la lave avec une solution aqueuse d'acide chlorhydrique 0,1N, puis une solution aqueuse saturée d'hydrogénocarbonate de sodium et une solution aqueuse saturée de chlorure de sodium. On sèche la phase organique sur sulfate de sodium, et on concentre le filtrat sous pression réduite, on purifie le résidu par chromatographie sur gel de silice en éluant avec un mélange 95/5 d'acétate d'éthyle et de méthanol, et on recristallise le solide obtenu dans l'acétate d'éthyle.
On obtient 6,5 g de produit pur.
Point de fusion : 148-150°C.
RMN ¹H : (DMSO) δ (ppm) 8, 15 (d, 1H) ; 7,75 (d, 1H); 7,4 (dd, 1H); 7,35 (d, 1H); 7,25 (m, 4H); 7,15 (m large 1H); 4,7.5 (t, 1H); 4, 35 (s, 2H); 4,25 (dd, 2H); 3, 95 (dd, 2H); 3,90 (s+m, 4H); 3,05 (m, 2H); 1,60 (m, 4H).

### Exemple 3 (Composé N°3).

*trans*-2-(5-phényl-1,3-dioxan-2-yl)éthylcarbamate de 2-(méthylamino)-2-oxoéthyle.

### 3.1. trans-[[[[2-(5-phényl-1,3-dioxan-2-yl)éthyl]amino] carbonyl]oxy]acétate d'éthyle.

On utilise la méthode de l'exemple 1.2. A partir de 1 g (4,8 mmoles) de *trans*-2-(5-phényl-1,3-dioxan-2-yl)éthanamine et 1,1 g (4,8 mmoles) de [(phénoxycarbonyl)oxy]acétate d'éthyle, on obtient 0,740 g d'ester sous forme d'huile.

### 3.2. trans-2-(5-phényl-1,3-dioxan-2-yl)éthylcarbamate de 2-(méthylamino)-2-oxoéthyle.

A une solution de 0,70 g (2,1 mmoles) de trans-[[[[2-(5-phényl-1,3-dioxan-2-yl)éthyl]amino]carbonyl]oxy]acétate d'éthyle, obtenu à l'étape 3.1, dans 4 ml de méthanol on ajoute, goutte à goutte, 3,3 ml (6,7 mmoles) d'une solution de méthylamine (2M dans le tétrahydrofurane) et on agite le mélange à température ambiante pendant 12 h. On concentre sous pression réduite, et on purifie le résidu par chromatographie sur gel de silice en éluant avec un mélange 90/10 de dichlorométhane et de méthanol. On triture l'huile obtenue dans le diisopropyléther et on obtient 0,450 g de produit pur.
Point de fusion : 89°C.
RMN ¹H : (CDCl₃) δ(ppm) 7,35-7,20 (m, 3H); 7,15 (dd, 2H); 6,15 (m large, 1H); 5,45 (m large, 1H); 4,75 (t, 1H); 4,60 (s, 2H); 4,20 (dd, 2H); 3,80 (dd, 2H); 3,40 (m, 2H); 3,20 (m, 1H); 2,85 (d, 3H); 1,90 (m, 2H).

### Exemple 4 (Composé N°63).

*trans*-3-[5-(6-méthoxynaphtalèn-1-yl)-1,3-dioxan-2-yl]propylcarbamate de 1*H*-imidazol-2-ylméthyle.

### 4.1. (1-triphénylméthyl-1H-imidazol-2-yl)méthylcarbonate de phényle.

On opère comme décrit dans l'exemple 1.1. A partir de 3 g (8,80 mmoles) de 1-triphénylméthyl-1*H*-imidazole-2-méthanol *(*J. Het. Chem., (1995), 32, 903-906) et 1,1 ml (8,80 mmoles) de chloroformiate de phényle, on obtient 3,9 g de produit qu'on utilise tel quel dans l'étape suivante.

### 4.2. trans-3-[5-(6-méthoxynaphtalèn-1-yl)-1,3-dioxan-2-yl]propylcarbamate de (1-triphénylméthyl-1H-imidazol-2-yl)méthyle.

On opère comme décrit dans l'exemple 1.2. A partir de 2,5 g (8,28 mmoles)de trans-3-[5-(6-méthoxynaphtalèn-1-yl)-1,3-dioxan-2-yl]propanamine et 3,8 g (8,28 mmoles) de (1-triphénylméthyl-1*H*-imidazol-2-yl)méthylcarbonate de phényle, obtenu à l'étape 4.1, on obtient 3,2 g d'un solide sous forme amorphe.

### 4.3. trans-3-[5-(6-méthoxynaphtalèn-1-yl)-1,3-dioxan-2-yl]propylcarbamate de 1H-imidazol-2-ylméthyle.

A une solution de 1,9 g (2,83 mmoles) de trans-3-[5-(6-méthoxynaphtalèn-1-yl)-1,3-dioxan-2-yl]propylcarbamate de (1-triphénylméthyl-1*H*-imidazol-2-yl)méthyle, obtenu à l'étape 4.2, dans 150 ml de dichlorométhane, on ajoute goutte à goutte, à température ambiante, une solution de 0,6 ml (2,83 mmoles) d'acide trifluoroacétique dans 2 ml de dichlorométhane et on agite le mélange à température ambiante pendant 12 h. On concentre sous pression réduite, on reprend le résidu avec du dichlorométhane et une solution aqueuse saturée d'hydrogénocarbonate de sodium, on sépare la phase organique, on la lave avec une solution aqueuse saturée de chlorure de sodium, on la sèche sur sulfate de sodium, on concentre sous pression réduite et on purifie le résidu par chromatographie sur gel de silice en éluant avec un mélange 98/2/0,2 de dichlorométhane, méthanol et ammoniaque.
Après recristallisation dans l'acétate d'éthyle on obtient finalement 0,820 g de produit pur.
Point de fusion : 130-132°C.
RMN ¹H : (CDCl₃) δ(ppm) 10,0 (m large, 1H); 8, 10. (d, 1H); 7,65 (d, 1H); 7,35 (dd, 1H); 7,25 (d, 1H); 7,15 (dd, 1H); 7,05 (dd, 1H); 7,00 (s, 2H); 5,15 (m+s, 3H); 4,70 (t, 1H); 4,30 (m, 2H); 3,95-3,90 (m, 6H); 3,30 (m, 2H); 1,85 (m, 4H).

### Exemple 5 (Composé N°46).

*trans*-3-[5-(4-chloronaphtalèn-1-yl)-1,3-dioxan-2-yl]propylcarbamate de 2-amino-2-oxoéthyle.

### 5.1 trans-3-[5-(4-chloronaphtalèn-1-yl)-1,3-dioxan-2-yl)-1-propanol.

A une solution de 1,18 g (5 mmoles) de 2-(4-chloronaphtalèn-1-yl)-1,3-propanediol dans 10 ml de dioxane, on ajoute 0,75 ml (10 mmoles) de 2,3-dihydrofurane puis 0,25 ml d'une solution d'acide chlorhydrique aqueuse concentrée (37%). On laisse réagir une nuit à température ambiante puis on ajoute 5 ml d'eau. On agite le mélange pendant 5 heures puis on le dilue dans 25 ml d'eau et 50 ml de dichlorométhane. On décante et on extrait la phase aqueuse par 50 ml de dichlorométhane. On lave les phases organiques par 25 ml d'une solution aqueuse saturée en chlorure de sodium, on sèche sur sulfate de sodium et on concentre sous pression réduite. On purifie le résidu par chromatographie sur gel de silice en éluant par un mélange 70/30 puis 60/40 de cyclohexane et d'acétate d'éthyle. Après recristallisation dans le diisopropyléther on obtient 0,557 g de produit sous forme de cristaux blancs.
Point de fusion : 127-129°C.

### 5.2 méthanesulfonate de trans-3-[5-(4-chloronaphtalèn-1-yl)-1,3-dioxan-2-yl]propyle.

A une solution de 0,530 g (1,72 mmoles) de *trans*-3-[5-(4-chloronaphtalèn-1-yl)-1,3-dioxan-2-yl]-1-propanol préparé à l'étape 5.1 et de 0,48 ml (3,45 mmoles) de triéthylamine dans 8 ml de dichlorométhane refroidie à 0°C sous atmosphère inerte, on ajoute goutte à goutte une solution de 0,256 g (2,23 mmoles) de chlorure de mésyle dans 2 ml de dichlorométhane. On agite le mélange réactionnel à 0°C pendant 1 heure. On ajoute 25 ml d'eau et 50 ml de dichlorométhane. On décante et on extrait la phase aqueuse par 50 ml de dichlorométhane. On lave les phases organiques par 25 ml d'une solution aqueuse saturée en chlorure de sodium, on séche sur sulfate de magnésium et on concentre sous vide pour obtenir 0,66 g de produit sous forme de solide blanc utilisé tel quel dans l'étape suivante.

### 5.3 trans-3-[3-(5-(4-chloronaphtalèn-1-yl)-1,3-dioxan-2-yl)propyl]-1,3-oxazolidine-2,4-dione.

On chauffe à reflux pendant une nuit sous atmosphère inerte, un mélange de 0,660 g (1,71 mmoles) de méthanesulfonate de trans-3-[5-(4-chloronaphtalèn-1-yl)-1,3-dioxan-2-yl]propyle obtenu à l'étape 5.2, de 0,208 g (2,05 mmoles) de 1,3-oxazolidine-2,4-dione (J.Med.Chem., 1991, 34, 1542-1543) et de 0,396 g (3,43 mmoles) de 1,1,3,3-tétraméthylguanidine dans 10 ml de tétrahydrofurane. On reprend dans 100 ml d'acétate d'éthyle et 25 ml d'eau. On décante. On lave la phase organique par 25 ml d'eau puis 25 ml d'une solution aqueuse saturée en chlorure de sodium. On réextrait les phases aqueuses par 50 ml d'acétate d'éthyle. On réunit les phases organiques, on sèche sur sulfate de sodium et on concentre sous pression réduite. On purifie le résidu par chromatographie sur gel de silice en éluant par un mélange 70/30 puis 60/40 de cyclohexane et d'acétate d'éthyle pour obtenir 0,483 g de produit sous forme de solide blanc.
Point de fusion : 125-127°C.

### 5.4 trans-3-[5-(4-chloronaphtalèn-1-yl)-1,3-dioxan-2-yl]propylcarbamate de 2-amino-2-oxoéthyle

On dissout 0,470 g (1,20 mmoles) de *trans*-3-[3-(5-(4-chloronaphtalèn-1-yl)-1,3-dioxan-2-yl)propyl]-1,3-oxazolidine-2,4-dione obtenu à l'étape 5.3 dans 3,5 ml de tetrahydrofurane et on ajoute 7 ml d'une solution d'ammoniaque 7N dans le méthanol. On laisse réagir une nuit à température ambiante puis on évapore à sec et on recristallise dans un mélange d'iso-propanol et de diisopropyléther pour obtenir 0,388 g de produit sous forme de cristaux blancs.
Point de fusion : 176-178°C.
LC-MS : M+H = 407
RMN ¹H (DMSO) δ (ppm): 8, 35 (d, 1H); 8,25 (d, 1H); 7,8 (m, 3H); 7,4 (d, 1H); 7,25 (m, 2H); 7,15 (s, 1H); 4,75 (t, 1H); 4,3 (s, 2H); 4,2 (m, 2H); 4,0-3,9 (m, 3H); 3,05 (t, 2H); 1,65 (m, 2H); 1,6 (m, 2H).

### Exemple 6 (Composé N°67)

trans-3-[5-(6-cyclopropylméthoxy-naphtalèn-1-yl)-1,3-dioxan-2-yl]propylcarbamate de 4-chlorophényle.

A une solution de 0,110 ml (0,78 mmoles) de chloroformiate de 4-chlorophényle et de 0,205 ml (1,2 mmoles) de *N,N-*diisopropyléthylamine dans 6 ml de dichlorométhane on ajoute par petite portion et à température ambiante 0,205 g (0,60 mmoles) de trans-3-[5-(6-cyclopropylméthoxy-naphtalèn-1-yl)-1,3-dioxan-2-yl]propylamine. On agite le mélange à température ambiante pendant 16 heures, puis on lave avec 5 ml d'une solution saturée de bicarbonate de sodium. On sépare les phases et on filtre la phase organique à travers un fritté hydrophobe. On concentre le filtrat sous pression réduite et on purifie le résidu par chromatographie sur gel de silice en éluant par un mélange 80/20 de cyclohexane et d'acétate d'éthyle. Après lavage dans 5 ml de diisopropyléther, on obtient 0,176 g de solide blanc.
LC-MS : M+H= 496
Point de fusion : 159-162°C
RMN ¹H : (CDCl₃) δ (ppm) : 8, 10 (d, 1H) ; 7,65 (d, 1H) ; 7, 45-7,20 (m, 4H); 7,20-7,00 (m, 4H); 5,30 (m large, 1H); 4,75 (t, 1H); 4,35 (dd, 2H); 4,10-3,80 (m, 5H); 3,50-3,25 (m, 2H); 1,95-1,70 (m, 4H) ; 1,45-1,20 (m, 1H) ; 0,80-0,65 (m, 2H); 0,50-0,30 (m, 2H).

### Exemple 7 (Composé N°68)

### trans-3-[5-(6-cyclopropylméthoxy-naphtalèn-1-yl)-1,3-dioxan-2-yl]-propylcarbamate de 2,2,2-trifluoroéthyle.

A une suspension de 0,205 g (1,01 mmoles) de chloroformiate de 4-nitrophényle et de 0,555 g (2,02 mmoles) de *N,N-*diisopropylaminoéthylpolystyrène (Ps-DIEA, 2% DVB, titre = 3,66 mmoles/g) dans 7,1 ml de dichlorométhane, on ajoute, goutte à goutte et à température ambiante 0,075 ml (1,01 mmoles) de 2,2,2-trifluoroéthanol. On agite le mélange sous agitation orbitalaire et à température ambiante pendant 16 heures. On filtre la résine à travers une cartouche munie d'un fritté et on rince avec 4 ml de dichlorométhane. On concentre le filtrat sous pression réduite et on reprend le résidu huileux ainsi obtenu dans 3,5 ml de 1,2-dichloroéthane. Successivement, on ajoute 0,134 ml (0,78 mmoles) de *N,N*-diisopropyléthylamine et 0,205 g (0,6 mmoles) de 3-[5-(6-cyclopropylméthoxy-naphtalèn-1-yl)-1,3-dioxan-2-yl]propylamine. On chauffe ce mélange réactionnel à 60°C pendant 16 heures. Après refroidissement, on lave avec 20 ml d'une solution de soude 1N. On sépare les phases et on filtre la phase organique à travers un fritté hydrophobe. On concentre le filtrat sous pression réduite et on purifie le résidu par chromatographie sur gel de silice en éluant par un mélange 80/20 de cyclohexane et d'acétate d'éthyle. Après lavage dans 5 ml de diisopropyléther, on obtient 0,076 g de solide blanc.
LC-MS : M+H= 468
Point de fusion : 105-107°C
RMN ¹H : (CDCl₃) δ (ppm) : 8,15 (d, 1H) ; 7,65 (d, 1H) ; 7,35 (dd, 1H); 7,25 (m, 1H); 7,15 (d, 1H); 7,10 (d, 1H); 5,15 (m large, 1H) ; 4,75 (t, 1H) ; 4,50 (q, 2H); 4,30 (dd, 2H) ; 4,10-3,80 (m, 5H); 3,40-3,20 (m, 2H); 1,90-1,65 (m, 4H); 1,45-1,20 (m, 1H); 0,75-0,60 (m, 2H); 0,50-0,35 (2H).

Le tableau qui suit illustre les structures chimiques et les propriétés physiques de quelques composés selon l'invention. Les composés du tableau présentent la configuration relative *trans* sur le cycle dioxane à l'exception des composés n°37 et 50 qui présente la configuration relative cis et les composés n°6, 9, 11, 13, 18, 20, 21 et 43 qui sont sous forme d'un mélange des stéréoisomères cis et trans. Tous les composés du tableau sont sous forme de bases.

**Tableau**

| | | | | | |
|---|---|---|---|---|---|
| 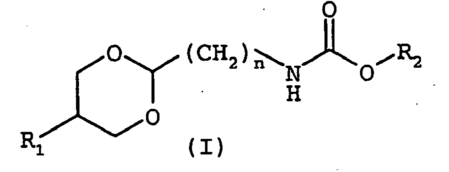 | | | | | |

| **N°** | **R₁** | **n** | **R₂** | **M+H** | **F (°C)** |
|---|---|---|---|---|---|
| 1. | phényl | 2 | CH₂CONH₂ | - | 172-174 |
| 2. | phényl | 3 | CH₂CONH₂ | - | 116-118 |
| 3. | phényl | 2 | CH₂CONHCH₃ | - | 89 |
| 4. | phényl | 3 | CH₂CONHCH₃ | - | 116 |
| 5. | phényl | 2 | CH₂CF₃ | 334 | 77-80 |
| 6. | phényl | 3 | CH₂CF₃ | 348 | 83-85 |
| 7. | phényl | 2 | phényl | 328 | 131-134 |
| 8. | phényl | 3 | phényl | 342 | 100-103 |
| 9. | phényl | 2 | 2-chlorophényl | 362 | 95-98 |
| 10. | phényl | 3 | 2-chlorophényl | 376 | 129-131 |
| 11. | phényl | 2 | 4-chlorophényl | 362 | 134-136 |
| 12. | phényl | 3 | 4-chlorophényl | 376 | 117-121 |
| 13. | phényl | 2 | 4-fluorophényl | 346 | 132-134 |
| 14. | phényl | 3 | 4-fluorophényl | 360 | 106-109 |
| 15. | phényl | 2 | 4-méthylphényl | 342 | 112-115 |
| 16. | phényl | 3 | 4-méthylphényl | 356 | 87-90 |
| 17. | phényl | 2 | 2-méthoxyphényl | 358 | - |
| 18. | phényl | 3 | 2-méthoxyphényl | 372 | 84-87 |
| 19. | phényl | 2 | 4-méthoxyphényl | 358 | 130-132 |
| 20. | phényl | 3 | 4-méthoxyphényl | 372 | 99-101 |
| 21. | phényl | 2 | 3-trifluorométhyl phényl | 396 | 87-90 |
| 22. | phényl | 3 | 3-trifluorométhyl phényl | 410 | 128-131 |
| 23. | 4-fluorophényl | 1 | 4-chlorophényl | - | 139-141 |
| 24. | 4-fluorophényl | 2 | CH₂CONHCH₃ | - | 124-126 |
| 25. | 4-fluorophényl | 3 | CH₂CONHCH₃ | - | 150-152 |
| 26. | 3-chlorophényl | 2 | 4-chlorophényl | - | 123-125 |
| 27. | 3-chlorophényl | 3 | 4-chlorophényl | - | 89-91 |
| 28. | 4-chlorophényl | 1 | 4-chlorophényl | - | 146-148 |
| 29. | 4-chlorophényl | 1 | CH₂CF₃ | - | 99-101 |
| 30. | 2-méthoxyphényl | 2 | 4-chlorophényl | - | 144-146 |
| 31. | 3-méthoxyphényl | 2 | 4-chlorophényl | - | 116-118 |
| 32. | 4-méthoxyphényl | 3 | 4-chlorophényl | - | 128-131 |
| 33. | 3-trifluorométhylphényl | 1 | 4-chlorophényl | - | 116-119 |
| 34. | 3-trifluorométhylphényl | 1 | CH₂CF₃ | - | 66-67 |
| 35. | 3-trifluorométhylphényl | 3 | 4-chlorophényl | - | 93-96 |
| 35. | 3-trifluorométhylphényl | 2 | CH₂CONHCH₃ | - | 118-120 |
| 37. | 3-trifluorométhylphényl | 2 | CH₂CONHCH₃ | - | 82-84 |
| 38. | naphtalèn-1-yl | 3 | CH₂CONH₂ | - | 112-114 |
| 39. | naphtalèn-1-yl | 2 | CH₂CONHCH₃ | - | 86-88 |
| 40. | naphtalén-1-yl | 3 | CH₂CONHCH₃ | - | 154 |
| 41. | naphtalèn-1-yl | 2 | CH₂CF₃ | 384 | 111-113 |
| 42. | naphtalèn-1-yl | 3 | CH₂CF₃ | 398 | 89-92 |
| 43. | naphtalèn-1-yl | 2 | CH₂-benzimidazol-2-yl | 432 | - |
| 44. | naphtalèn-1-yl | 2 | phényl | 378 | 131-133 |
| 45. | naphtalèn-1-yl | 3 | phényl | 392 | 125-127 |
| 46. | 4-chlôronaphtalèn-1-yl | 3 | CH₂CONH₂ | 407 | 176-178 |
| 47. | 4-chloronaphtalèn-1-yl | 3 | CH₂CONHCH₃ | - | 190-192 |
| 48. | 6-chloronaphtalèn-1-yl | 3 | CH₂CONHCH₃ | - | 182-184 |
| 49. | 6-méthoxynaphtalèn-1-yl | 3 | CH₂CONH₂ | - | 148-150 |
| 50. | 6-méthoxynaphtalèn-1-yl | 3 | CH₂CONH₂ | - | 144-147 |
| 51. | 6-méthoxynaphtalèn-1-yl | 1 | CH₂CONHCH₃ | - | 194-196 |
| 52. | 6-méthoxynaphtalèn-1-yl | 1 | 4-chlorophényl | - | 133-136 |
| 53. | 6-méthoxynaphtalèn-1-yl | 1 | CH₂CF₃ | - | 142-144 |
| 54. | 6-méthoxynaphtalèn-1-yl | 2 | CH₂CONHCH₃ | - | 136-138 |
| 55. | 6-méthoxynaphtalèn-1-yl | 2 | 4-chlorophényl | - | 129-131 |
| 56. | 6-méthoxynaphtalèn-1-yl | 2 | CH₂CF₃ | - | 93-95 |
| 57. | 6-méthoxynaphtalèn-1-yl | 3 | CH₂CONHCH₃ | - | 128-130 |
| 58. | 6-méthoxynaphtalèn-1-yl | 3 | CH₂CONHCH₂CH₃ | - | 170-172 |
| 59. | 6-méthoxynaphtalèn-1-yl | 3 | CH (CH₃)CONHCH₃ | - | 154 |
| 60. | 6-méthoxynaphtalèn-1-yl | 3 | CH₂CONHCH₂-pyridin-4-yl | - | 152 |
| 61. | 6-méthoxynaphtalèn-1-yl | 3 | CH₂CONH-cyclopropyle | - | 176 |
| 62. | 6-méthoxynaphtalèn-1-yl | 3 | CH₂CF₃ | - | 111 |
| 63. | 6-méthoxynaphtalèn-1-yl | 3 | CH₂-imidazol-2-yl | - | 130-132 |
| 64. | 6-méthoxynaphtalèn-1-yl | 3 | CH₂-benzimidazol-2-yl - | | 175-176 |
| 65. | 6-méthoxynaphtalèn-1-yl | 3 | phényl | - | 128 |
| 66. | 6-cyclopropylméthoxynaphtalèn-1-yl | 3 | CH₂CONH₂ | - | 137-139 |
| 67. | 6-cyclopropylméthoxynaphtalèn-1-yl | 3 | 4-chlorophényl | 496 | 159-162 |
| 68. | 6-cyclopropylméthoxynaphtalèn-1-yl | 3 | CH₂CF₃ | 468 | 105-107 |
| 69. | 6-phénylméthoxynaphtalèn-1-yl | 3 | CH₂CONH₂ | - | 154-156 |
| 70. | 6-hydroxynaphtalèn-1-yl | 3 | CH₂CONH₂ | - | 166-170 |
| 71. | 6-hydroxynaphtalèn-1-yl | 3 | CH₂CONHCH₃ | - | 140-148 |
| 72. | 7-méthoxynaphtalèn-1-yl | 3 | CH₂CONH₂ | - | 156-158 |
| 73. | 7-méthoxynaphtalèn-1-yl | 3 | CH₂CONHCH₃ | - | 144-146 |
| 74. | 7-méthoxynaphtalèn-1-yl | 3 | CH₂CF₃ | 428 | 93-96 |
| 75. | 7-méthoxynaphtalèn-1-yl | 3 | phényl | 422 | 151-153 |
| 76. | naphtalèn-2-yl | 3 | phényl | 392 | 130-131 |
| 77. | naphtalèn-2-yl | 3 | CH₂CONHCH₃ | - | 144-146 |
| 78. | naphtalèn-2-yl | 3 | CH₂CF₃ | 398 | 130-132 |

Les composés de l'invention ont fait l'objet d'essais pharmacologiques permettant de déterminer leur effet inhibiteur de l'enzyme FAAH (Fatty Acid Amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimented Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide non radiomarquée et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est compté par scintillation liquide.
Dans ces conditions, les composés les plus actifs de l'invention présentent des CI₅₀ (concentration inhibant de 50% l'activité enzymatique contrôle de la FAAH) comprises entre 0, 005 et 1 µM.

Il apparaît donc que les composés selon l'invention ont une activité inhibitrice sur l'enzyme FAAH.

L'activité *in vivo* des composés de l'invention a été évaluée dans un test d'analgésie.
Ainsi, l'administration intrapéritonéale (i.p.) de PBQ (phénylbenzoquinone, 2 mg/kg dans une solution de NaCl à 0,9% contenant 5% d'éthanol) chez des souris males OF1 de 25 à 30 g, provoque des étirements abdominaux, en moyenne 30 torsions ou contractions pendant la période de 5 à 15 minutes après injection. Les composés testés sont administrés par voie orale ou par voie i.p. en suspension dans du Tween 80 à 0,5%, 30 minutes, 60 minutes ou 120 minutes avant l'administration de PBQ. Dans ces conditions, les composés les plus puissants de l'invention réduisent de 50 à 70 % le nombre d'étirements induits par le PBQ, dans une gamme de doses comprise entre 1 et 30 mg/kg.

L'enzyme FAAH *(*Chemistry and Physics of Lipids, (2000), 108, 107-121) catalyse l'hydrolyse des dérivés endogènes d'amides et d'esters de différents acides gras tels que la N-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement de toute pathologie dans laquelle les cannabinoides endogènes et/ou tout autre substrat métabolisé par l'enzyme FAAH, sont impliqués.
On peut par exemple citer les maladies et les affections suivantes :
la douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ;
les maladies neuro-dégénératives aiguës ou chroniques : maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies autoimmunes: psoriasis, lupus érythémateux, maladies du tissu conjonctif ou connectivites, syndrome de Sjögren's, spondylarthrite ankylosante, spondylarthrite indifférenciée, maladie de Behcet's, anémies autoimmunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires, virales ou bactériennes : SIDA, méningites
les maladies inflammatoires, notamment les maladies articulaires : arthrite, arthrite rhumatoïde, ostéoarthrite, spondylite, goutte, vascularite, maladie de Crohn, syndrome du colon irritable ;
l'ostéoporose ;
les affections oculaires : hypertension oculaire, glaucome ; les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées, reflux gastrooesophagien ;
l'incontinence urinaire et l'inflammation vésicale.

L'utilisation des composés selon l'invention pour la préparation d'un médicament destiné à prévenir ou à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans la prévention et le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prévention ou le traitement des troubles ou des maladies ci-dessus mentionnées.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire, intraveineuse ou intrathécale et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) 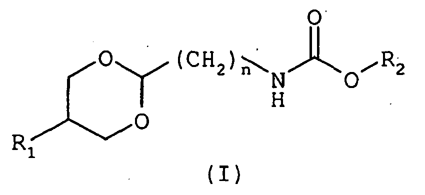 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ;
R₂ représente
soit un groupe de formule générale CHR₃CONHR₄ dans laquelle
R₃ représente un atome d'hydrogène ou un groupe méthyle et R₄ représente un atome d'hydrogène ou un groupe C₁₋₃-alkyle, C₃₋₅-cycloalkyle ou (pyridin-4-yl)méthyle,
soit un groupe 2,2,2-trifluoroéthyle,
soit un groupe (imidazol-2-yl)méthyle,
soit un groupe (benzimidazol-2-yl)méthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy ; et
n représente un nombre allant de 1 à 3 ;
à l'état de base, de sel d'addition à un acide, d'hydrate ou de solvat.

2. Composé de formule (I) selon la revendication 1, **caractérisé en ce que** R₁ représente un groupe naphtalènyle, éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy; à l'état de base, de sel d'addition à un acide, d'hydrate ou de solvat.

3. Composé de formule (I) selon la revendication 1, **caractérisé en ce que** R₂ représente
soit un groupe de formule générale CHR₃CONHR₄ dans laquelle
R₃ représente un atome d'hydrogène et
R₄ représente un atome d'hydrogène ou un groupe C₁₋₃-alkyle, ou (pyridin-4-yl)méthyle,
soit un groupe 2,2,2-trifluoroéthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy; à l'état de base, de sel d'addition à un acide, d'hydrate ou de solvat.

4. Composé de formule (I) selon la revendication 1, **caractérisé en ce que** n représente 2 ou 3; à l'état de base, de sel d'addition à un acide, d'hydrate ou de solvat.

5. Composé de formule (I) selon l'une quelconque des revendications 1 à 4 choisi parmi :
*trans*-3-[5-(naphtalèn-1-yl)-1,3-dioxan-2-yl]propylcarbamate de 2-amino-2-oxoéthyle ;
*trans*-2-[5-(naphtalèn-1-yl)-1,3-dioxan-2-yl]éthylcarbamate de 2-(méthylamino)-2-oxoéthyle ;
*trans*-3-[5-(naphtalèn-1-yl)-1,3-dioxan-2-yl]propylcarbamate de 2-(méthylamino)-2-oxoéthyle ;
*trans*-2-[5-(naphtalèn-1-yl)-1,3-dioxan-2-yl]éthylcarbamate de 2,2,2-trifluoroéthyle ;
*trans*-3-[5-(naphtalèn-1-yl)-1,3-dioxan-2-yl]propylcarbamate de 2,2,2-trifluoroéthyle ;
*trans*-2-[5-(naphtalèn-1-yl)-1,3-dioxan-2-yl]éthylcarbamate de phényle ;
*trans*-3-[5-(naphtalèn-1-yl)-1,3-dioxan-2-yl]propylcarbamate de phényle ;
*trans*-3-[5-(4-chloronaphtalèn-1-yl)-1,3-dioxan-2-yl]propylcarbamate de 2-amino-2-oxoéthyle ;
*trans*-3-[5-(4-chloronaphtalèn-1-yl)-1,3-dioxan-2-yl]propylcarbamate de 2-(méthylamino)-2-oxoéthyle ;
*trans*-3-[5-(6-chloronaphtalèn-1-yl)-1,3-dioxan-2-yl]propylcarbamate de 2-(méthylamino)-2-oxoéthyle ;
*trans*-3-[5-(6-méthoxynaphtalèn-1-yl)-1,3-dioxan-2-yl]propylcarbamate de 2-amino-2-oxoéthyle ;
*cis*-3-[5-(6-méthoxynaphtalèn-1-yl)-1,3-dioxan-2-yl]propylcarbamate de 2-amino-2-oxoéthyle ;
*trans*-2-[5-(6-méthoxynaphtalèn-1-yl)-1,3-dioxan-2-yl]éthylcarbamate de 2-(méthylamino)-2-oxoéthyle ;
*trans*-2-[5-(6-méthoxynaphtalèn-1-yl)-1,3-dioxan-2-yl]éthylcarbamate de 4-chlorophényle ;
*trans*-2-[5-(6-méthoxynaphtalèn-1-yl)-1,3-dioxan-2-yl]éthylcarbamate de 2,2,2-trifluoroéthyle ;
*trans-*3-[5-(6-méthoxynaphtalèn-1-yl)-1,3-dioxan-2-yl]propylcarbamate de 2-(méthylamino)-2-oxoéthyle ;
*trans*-3-[5-(6-méthoxynaphtalèn-1-yl)-1,3-dioxan-2-yl]propylcarbamate de 2-(éthylamino)-2-oxoéthyle ;
*trans*-3-[5-(6-méthoxynaphtalèn-1-yl)-1,3-dioxan-2-yl]propylcarbamate de 2-[(pyridin-4-yl)méthylamino]-2-oxoéthyle ;
*trans*-3-[5-(6-méthoxynaphtalèn-1-yl)-1,3-dioxan-2-yl]propylcarbamate de 2,2,2-trifluoroéthyle ;
*trans*-3-[5-(6-méthoxynaphtalèn-1-yl)-1,3-dioxan-2-yl]propylcarbamate de phényle ;
*trans*-3-[5-(6-cyclopropylméthoxynaphtalèn-1-yl)-1,3-dioxan-2-yl]propylcarbamate de 2-amino-2-oxoéthyle ;
*trans*-3-[5-(6-cyclopropylméthoxynaphtalèn-1-yl)-1,3-dioxan-2-yl]propylcarbamate de 4-chlorophényle ;
*trans*-3-[5-(6-cyclopropylméthoxynaphtalèn-1-yl)-1,3-dioxan-2-yl]propylcarbamate de 2,2,2-trifluoroéthyle ;
*trans*-3-[5-(6-phénylméthoxynaphtalèn-1-yl)-1,3-dioxan-2-yl]propylcarbamate de 2-amino-2-oxoéthyle ;
*trans*-3-[5-(6-hydroxynaphtalèn-1-yl)-1,3-dioxan-2-yl]propylcarbamate de 2-amino-2-oxoéthyle ;
*trans*-3-[5-(6-hydroxynaphtalèn-1-yl)-1,3-dioxan-2-yl]propylcarbamate de 2-(méthylamino)-2-oxoéthyle ;
*trans*-3-[5-(7-méthoxynaphtalèn-1-yl)-1,3-dioxan-2-yl]propylcarbamate de 2-amino-2-oxoéthyle ;
*trans*-3-[5-(7-méthoxynaphtalèn-1-yl)-1,3-dioxan-2-yl]propylcarbamate de 2-(méthylamino)-2-oxoéthyle ;
*trans*-3-[5-(7-méthoxynaphtalèn-1-yl)-1,3-dioxan-2-yl]propylcarbamate de 2,2,2-trifluoroéthyle ;
*trans*-3-[5-(7-méthoxynaphtalèn-1-yl)-1,3-dioxan-2-yl]propylcarbamate de phényle ;
*trans*-3-[5-(naphtalèn-2-yl)-1,3-dioxan-2-yl]propylcarbamate de phényle
*trans*-3-[5-(naphtalèn-2-yl)-1,3-dioxan-2-yl]propylcarbamate de 2-(méthylamino)-2-oxoéthyle ;
*trans*-3-[5-(naphtalèn-2-yl)-1,3-dioxan-2-yl]propylcarbamate de 2,2,2-trifluoroéthyle ;
à l'état de base, de sel d'addition à un acide, d'hydrate ou de solvat.

6. Procédé de préparation d'un composé de formule (I), 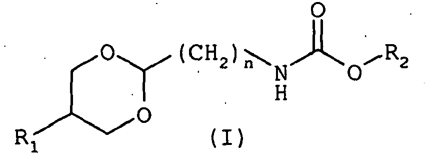 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ;
R₂ représente un groupe de formule générale CHR₃CONHR₄ dans laquelle R₃ représente un atome d'hydrogène ou un groupe méthyle et R₄ représente un atome d'hydrogène ou un groupe C₁₋₃-alkyle, C₃₋₅-cycloalkyle ou (pyridin-4-yl)méthyle ; et
n représente un nombre allant de 1 à 3 ;
comprenant l'étape consistant à
transformer le carbamate-ester de formule générale (Ia) 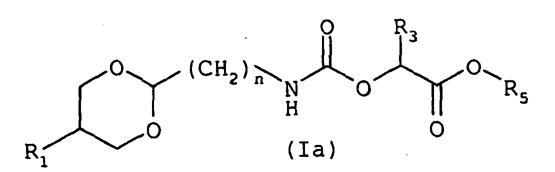 dans laquelle R₁, R₃ et n sont tels que définis dans la formule générale (I) et R₅ représente un groupe méthyle ou éthyle,
en composé de formule générale (I), soit par aminolyse directe au moyen d'une amine de formule générale R₄NH₂ dans laquelle R₄ est tel que défini dans la formule générale (I), soit par hydrolyse en acide de formule générale (Ia), dans laquelle R₅ représente un atome d'hydrogène, suivie d'un couplage avec une amine de formule générale R₄NH₂ dans laquelle R₄ est tel que défini dans la formule générale (I).

7. Procédé de préparation d'un composé de formule (I), 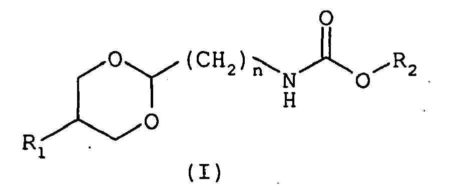 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ;
R₂ représente un groupe de formule générale CHR₃CONHR₄ dans laquelle R₃ représente un atome d'hydrogène ou un groupe méthyle et R₄ représente un atome d'hydrogène ou un groupe C₁₋₃-alkyle, C₃₋₅-cycloalkyle ou (pyridin-4-yl)méthyle ; et
n représente un nombre allant de 1 à 3 ;
comprenant l'étape consistant à
transformer le dérivé oxazolidine-dione de formule générale (VI) 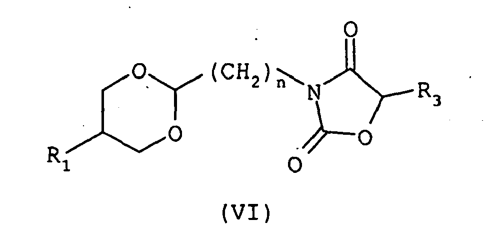 dans laquelle R₁, R₃ et n sont tels que définis dans la formule générale (I),
en composé de formule générale (I) par aminolyse au moyen d'une amine de formule générale R₄NH₂ où R₄ est tel que défini dans la formule générale (I).

8. Procédé de préparation d'un composé de formule (I), 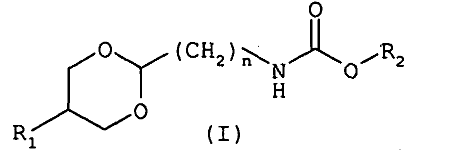 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy, C₃₋₆-cycloalkyl-O- ou C₃₋₆-cycloalkylC₁₋₃-alcoxy ;
R₂ représente
soit un groupe de formule générale CHR₃CONHR₄ dans laquelle
R₃ représente un atome d'hydrogène ou un groupe méthyle et
R₄ représente un atome d'hydrogène ou un groupe C₁₋₃-alkyle, C₃₋₅-cycloalkyle ou (pyridin-4-yl)méthyle,
soit un groupe 2,2,2-trifluoroéthyle,
soit un groupe (imidazol-2-yl)méthyle,
soit un groupe (benzimidazol-2-yl)méthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy ; et
n représente un nombre allant de 1 à 3 ;
comprenant l'étape consistant à
faire réagir une amine de formule générale (II), 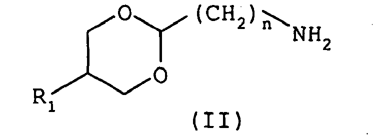 dans laquelle R₁ et n sont tels que définis dans la formule générale (I), avec un carbonate de formule générale (III), 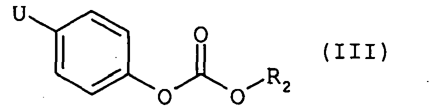 dans laquelle U représente un atome d'hydrogène ou un groupement nitro et R₂ est tel que défini dans la formule générale (I).

9. Composé répondant à la formule générale (Ia), 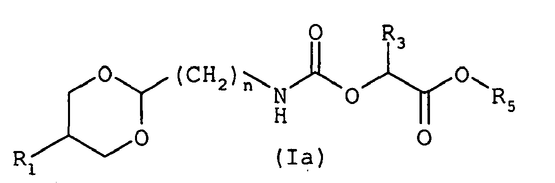 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy ou C₃₋₆-cycloalkylC₁₋₃-alcoxy ;
R₃ représente un atome d'hydrogène ou un groupe méthyle ;
R₅ représente un atome d'hydrogène, un groupe méthyle ou éthyle ; et
n représente un nombre allant de 1 à 3.

10. Composé répondant à la formule générale (VI) 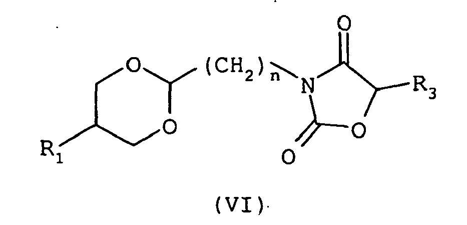 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃-alcoxy, trifluorométhyle, trifluorométhoxy, benzyloxy ou C₃₋₆-cycloalkylC₁₋₃-alcoxy ;
R₃ représente un atome d'hydrogène ou un groupe méthyle ; et n représente un nombre allant de 1 à 3.

11. Composition pharmaceutique comprenant au moins un composé de formule (I) selon l'une quelconque des revendications 1 à 5, à l'état de base, de sel, d'hydrate ou de solvat pharmaceutiquement acceptable et éventuellement un ou plusieurs excipients pharmaceutiquement acceptables.

12. Composé de formule (I) selon l'une quelconque des revendications 1 à 5, à l'état de base, de sel, d'hydrate ou de solvat pharmaceutiquement acceptable, pour son utilisation comme médicament.

13. Utilisation d'un composé de formule (I) selon l'une quelconque des revendications 1 à 5, à l'état de base, de sel, d'hydrate ou de solvat pharmaceutiquement acceptable, pour la préparation d'un médicament destiné à prévenir ou à traiter une pathologie dans laquelle les cannabinoides endogènes et/ou tout autre substrat métabolisé par l'enzyme FAAH, sont impliqués.

14. Utilisation d'un composé de formule (I) selon la revendication 13, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales ou l'incontinence urinaire.

15. Utilisation selon la revendication 14 **caractérisée en ce que** les douleurs aiguës ou chroniques sont de type neurogène, périphériques ou associées aux maladies inflammatoires.

16. Utilisation selon la revendication 14 **caractérisée en ce que** les troubles du comportement alimentaire correspondent aux anorexies et cachexies.

17. Utilisation selon la revendication 14 **caractérisée en ce que** les pathologies neurologiques et psychiatriques sont choisies parmi les pathologies suivantes : tremblements, dyskinésies, dystonies, spasticité, comportements compulsifs et obsessionnels, syndrome de Tourette, dépression, anxiété, troubles de l'humeur, psychoses.

18. Utilisation selon la revendication 14 **caractérisée en ce que** les maladies neuro-dégénératives aiguës et chroniques sont choisies parmi les maladies suivantes : maladie de Parkinson, maladie d'Alzheimer, démence sénile, chorée de Huntington, lésions liées à l'ischémie cérébrale et aux traumatismes crâniens et médullaires.

19. Utilisation selon la revendication 14 **caractérisée en ce que** les maladies cardiovasculaires sont choisies parmi les maladies suivantes : hypertension, arythmies cardiaques, artériosclérose, crise cardiaque, ischémies cardiaques.

20. Utilisation selon la revendication 14 **caractérisée en ce que** les cancers sont choisis parmi les tumeurs bénignes de la peau, les papillomes, les tumeurs de la prostate et les tumeurs cérébrales.

21. Utilisation selon la revendication 14 **caractérisée en ce que** les désordres du système immunitaire sont les maladies auto-immunes.

22. Utilisation selon la revendication 14 **caractérisée en ce que** les maladies allergiques sont choisies parmi : l'hypersensibilité immédiate ou retardée, les rhinites ou conjonctivites allergiques, les dermatites de contact.

23. Utilisation selon la revendication 14 **caractérisée en ce que** les maladies inflammatoires sont les maladies articulaires.

24. Utilisation selon la revendication 14 **caractérisée en ce que** les affections pulmonaires sont choisies parmi : les maladies des voies respiratoires, les bronchospasmes, la toux, l'asthme, la bronchite chronique, l'obstruction chronique des voies respiratoires, l'emphysème.

25. Utilisation selon la revendication 14 **caractérisée en ce que** les maladies gastro-intestinales sont choisies parmi : le syndrome du colon irritable, les désordres inflammatoires intestinaux, les ulcères, les diarrhées, le reflux gastrooesophagien.

## Claims

1. Compound corresponding to formula (I) 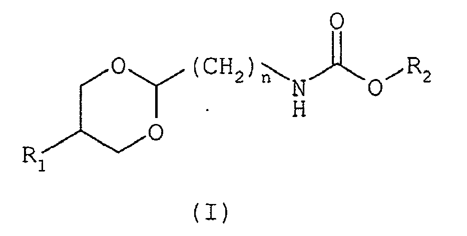 in which
R₁ represents a phenyl or naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy, (C₃-C₆)cycloalkyl-O- or (C₃-C₆)cycloalkyl(C₁-C₃)alkoxy groups;
R₂ represents
either a group of general formula CHR₃CONHR₄ in which
R₃ represents a hydrogen atom or a methyl group and
R₄ represents a hydrogen atom or a (C₁-C₃)alkyl, (C₃-C₅)cycloalkyl or (pyridin-4-yl)methyl group,
or a 2,2,2-trifluoroethyl group,
or an (imidazol-2-yl)methyl group,
or a (benzimidazol-2-yl)methyl group,
or a phenyl group optionally substituted with one or more halogen atoms or cyano, nitro, (C₁-C₃)alkyl, (C₁-C₃)alkoxy, trifluoromethyl or trifluoromethoxy groups; and
n represents a number ranging from 1 to 3;
in the form of a base, of an addition salt with an acid, of a hydrate or of a solvate.

2. Compound of formula (I) according to Claim 1, **characterized in that** R₁ represents a naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy, (C₃-C₆)cycloalkyl-O- or (C₃-C₆)-cycloalkyl(C₁-C₃)alkoxy groups, in the form of a base, of an addition salt with an acid, of a hydrate or of a solvate.

3. Compound of formula (I) according to Claim 1, **characterized in that** R₂ represents
either a group of general formula CHR₃CONHR₄ in which
R₃ represents a hydrogen atom and
R₄ represents a hydrogen atom or a (C₁-C₃)alkyl or (pyridin-4-yl)methyl group,
or a 2,2,2-trifluoroethyl group,
or a phenyl group optionally substituted with one or more halogen atoms or cyano, nitro, (C₁-C₃)alkyl, (C₁-C₃)alkoxy, trifluoromethyl or trifluoromethoxy groups, in the form of a base, of an addition salt with an acid, of a hydrate or of a solvate.

4. Compound of formula (I) according to Claim 1, **characterized in that** n represents 2 or 3, in the form of a base, of an addition salt with an acid, of a hydrate or of a solvate.

5. Compound of formula (I) according to any one of Claims 1 to 4, chosen from:
2-amino-2-oxoethyl trans-3-[5-(naphthalen-1-yl)-1,3-dioxan-2-yl]propylcarbamate;
2-(methylamino)-2-oxoethyl trans-2-[5-(naphthalen-1-yl)-1,3-dioxan-2-yl]ethylcarbamate;
2-(methylamino)-2-oxoethyl trans-3-[5-(naphthalen-1-yl)-1,3-dioxan-2-yl]propylcarbamate;
2,2,2-trifluoroethyl *trans*-2-[5-(naphthalen-1-yl)-1,3-dioxan-2-yl]ethylcarbamate;
2,2,2-trifluoroethyl *trans*-3-[5-(naphthalen-1-yl)-1,3-dioxan-2-yl]propylcarbamate;
phenyl *trans*-2-[5-(naphthalen-1-yl)-1,3-dioxan-2-yl]ethylcarbamate;
phenyl *trans*-3-[5-(naphthalen-1-yl)-1,3-dioxan-2-yl]propylcarbamate;
2-amino-2-oxoethyl *trans*-3-[5-(4-chloronaphthalen-1-yl)-1,3-dioxan-2-yl]propylcarbamate;
2-(methylamino)-2-oxoethyl *trans*-3-[5-(4-chloronaphthalen-1-yl)-1,3-dioxan-2-yl]propylcarbamate;
2-(methylamino)-2-oxoethyl *trans*-3-[5-(6-chloronaphthalen-1-yl)-1,3-dioxan-2-yl]propylcarbamate;
2-amino-2-oxoethyl *trans*-3-[5-(6-methoxynaphthalen-1-yl)-1,3-dioxan-2-yl]propylcarbamate;
2-amino-2-oxoethyl cis-3-[5-(6-methoxynaphthalen-1-yl)-1,3-dioxan-2-yl]propylcarbamate;
2-(methylamino)-2-oxoethyl *trans*-2-[5-(6-methoxynaphthalen-1-yl)-1,3-dioxan-2-yl]ethylcarbamate;
4-chlorophenyl *trans*-2-[5-(6-methoxynaphthalen-1-yl)-1,3-dioxan-2-yl]ethylcarbamate;
2,2,2-trifluoroethyl *trans*-2-[5-(6-methoxynaphthalen-1-yl)-1,3-dioxan-2-yl]ethylcarbamate;
2-(methylamino)-2-oxoethyl *trans*-3-[5-(6-methoxynaphthalen-1-yl)-1,3-dioxan-2-yl]propylcarbamate;
2-(ethylamino)-2-oxoethyl *trans*-3-[5-(6-methoxynaphthalen-1-yl)-1,3-dioxan-2-yl]propylcarbamate;
2-[(pyridin-4-yl)methylamino]-2-oxoethyl *trans*-3-[5-(6-methoxynaphthalen-1-yl)-1,3-dioxan-2-yl]propylcarbamate;
2,2,2-trifluoroethyl *trans*-3-[5-(6-methoxynaphthalen-1-yl)-1,3-dioxan-2-yl]propylcarbamate;
phenyl *trans*-3-[5-(6-methoxynaphthalen-1-yl)-1,3-dioxan-2-yl]propylcarbamate;
2-amino-2-oxoethyl *trans*-3-[5-(6-cyclopropylmethoxynaphthalen-1-yl)-1,3-dioxan-2-yl]propylcarbamate;
4-chlorophenyl *trans*-3-[5-(6-cyclopropylmethoxynaphthalen-1-yl)-1,3-dioxan-2-yl]propylcarbamate;
2,2,2-trifluoroethyl *trans*-3-[5-(6-cyclopropylmethoxynaphthalen-1-yl)-1,3-dioxan-2-yl]propylcarbamate;
2-amino-2-oxoethyl *trans*-3-[5-(6-phenylmethoxynaphthalen-1-yl)-1,3-dioxan-2-yl]propylcarbamate;
2-amino-2-oxoethyl *trans*-3-[5-(6-hydroxynaphthalen-1-yl)-1,3-dioxan-2-yl]propylcarbamate;
2-(methylamino)-2-oxoethyl *trans*-3-[5-(6-hydroxynaphthalen-1-yl)-1,3-dioxan-2-yl]propylcarbamate;
2-amino-2-oxoethyl *trans*-3-[5-(7-methoxynaphthalen-1-yl)-1,3-dioxan-2-yl]propylcarbamate;
2-(methylamino)-2-oxoethyl *trans*-3-[5-(7-methoxynaphthalen-1-yl)-1,3-dioxan-2-yl]propylcarbamate;
2,2,2-trifluoroethyl *trans*-3-[5-(7-methoxynaphthalen-1-yl)-1,3-dioxan-2-yl]propylcarbamate;
phenyl *trans*-3-[5-(7-methoxynaphthalen-1-yl)-1,3-dioxan-2-yl]propylcarbamate;
phenyl *trans*-3-[5-(naphthalen-2-yl)-1,3-dioxan-2-yl]propylcarbamate;
2-(methylamino)-2-oxoethyl *trans*-3-[5-(naphthalen-2-yl)-1,3-dioxan-2-yl]propylcarbamate;
2,2,2-trifluoroethyl *trans*-3-[5-(naphthalen-2-yl)-1,3-dioxan-2-yl]propylcarbamate;
in the form of a base, of an addition salt with an acid, of a hydrate or of a solvate.

6. Method for preparing a compound of formula (I), 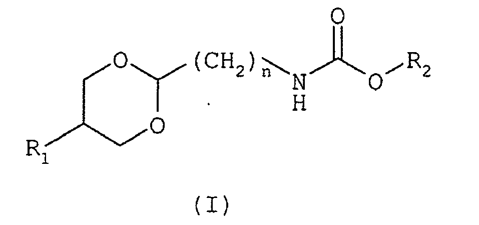 in which
R₁ represents a phenyl or naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy, (C₃-C₆)cycloalkyl-O- or (C₃-C₆)cycloalkyl(C₁-C₃)alkoxy groups;
R₂ represents a group of general formula CHR₃CONHR₄ in which R₃ represents a hydrogen atom or a methyl group and R₄ represents a hydrogen atom or a (C₁-C₃)alkyl, (C₃-C₅)cycloalkyl or (pyridin-4-yl)methyl group; and
n represents a number ranging from 1 to 3;
comprising the step consisting in
converting the carbamate ester of general formula (Ia) 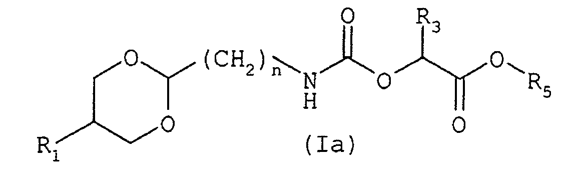 in which R₁, R₃ and n are as defined in general formula (I) and R₅ represents a methyl or ethyl group, to a compound of general formula (I), either by direct aminolysis by means of an amine of general formula R₄NH₂ in which R₄ is as defined in general formula (I), or by hydrolysis to an acid of general formula (Ia), in which R₅ represents a hydrogen atom, followed by coupling with an amine of general formula R₄NH₂ in which R₄ is as defined in general formula (I).

7. Method for preparing a compound of formula (I), 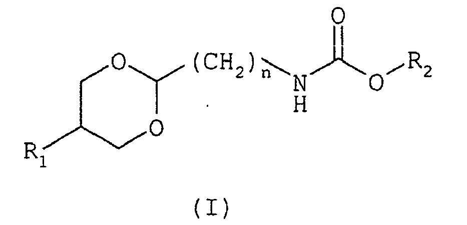 in which
R₁ represents a phenyl or naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy, (C₃-C₆)cycloalkyl-O- or (C₃-C₆)cycloalkyl(C₁-C₃)alkoxy groups;
R₂ represents a group of general formula CHR₃CONHR₄ in which R₃ represents a hydrogen atom or a methyl group and R₄ represents a hydrogen atom or a (C₁-C₃)alkyl, (C₃-C₅)cycloalkyl or (pyridin-4-yl)methyl group; and
n represents a number ranging from 1 to 3;
comprising the step consisting in
converting the oxazolidinedione derivative of general formula (VI) 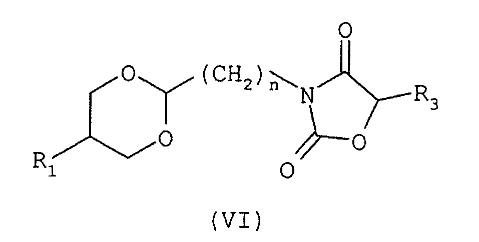 in which R₁, R₃ and n are as defined in general formula (I),
to a compound of general formula (I) by aminolysis by means of an amine of general formula R₄NH₂ where R₄ is as defined in general formula (I).

8. Method for preparing a compound of formula (I), 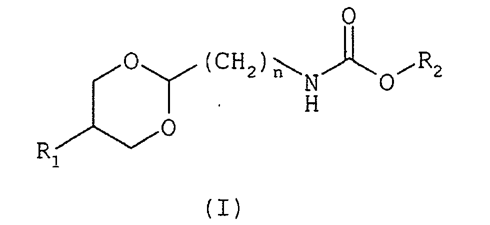 in which
R₁ represents a phenyl or naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy, (C₃-C₆)cycloalkyl-O- or (C₃-C₆)cycloalkyl(C₁-C₃)alkoxy groups;
R₂ represents
either a group of general formula CHR₃CONHR₄ in which
R₃ represents a hydrogen atom or a methyl group and
R₄ represents a hydrogen atom or a (C₁-C₃)alkyl, (C₃-C₅)cycloalkyl or (pyridin-4-yl)methyl group,
or a 2,2,2-trifluoroethyl group,
or an (imidazol-2-yl)methyl group,
or a (benzimidazol-2-yl)methyl group,
or a phenyl group optionally substituted with one or more halogen atoms or cyano, nitro, (C₁-C₃)alkyl, (C₁-C₃)alkoxy, trifluoromethyl or trifluoromethoxy groups; and
n represents a number ranging from 1 to 3;
comprising the step consisting in
reacting an amino of general formula (II), 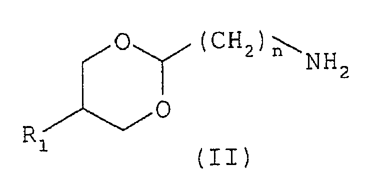 in which R₁ and n are as defined in general formula (I), with a carbonate of general formula (III), 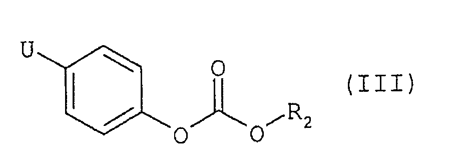 in which U represents a hydrogen atom or a nitro group and R₂ is as defined in general formula (I).

9. Compound corresponding to general formula (Ia) 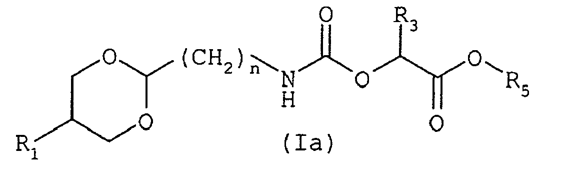 in which
R₁ represents a phenyl or a naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy or (C₃-C₆)cycloalkyl(C₁-C₃)alkoxy groups;
R₃ represents a hydrogen atom or a methyl group;
R₅ represents a hydrogen atom, or a methyl or ethyl group; and
n represents a number ranging from 1 to 3.

10. Compound corresponding to general formula (VI) 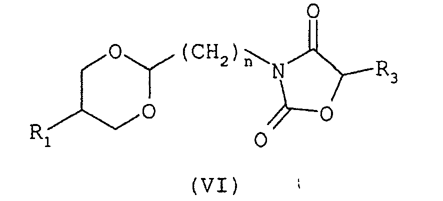 in which
R₁ represents a phenyl or naphthalenyl group optionally substituted with one or more halogen atoms or hydroxyl, cyano, nitro, (C₁-C₃)alkyl, (C₁-C₃)alkoxy, trifluoromethyl, trifluoromethoxy, benzyloxy or (C₃-C₆)cycloalkyl(C₁-C₃)alkoxy groups;
R₃ represents a hydrogen atom or a methyl group; and
n represents a number ranging from 1 to 3.

11. Pharmaceutical composition comprising at least one compound of formula (I) according to any one of Claims 1 to 5, in the form of a base, of a salt, of a hydrate or of a solvate which is pharmaceutically acceptable and, optionally, one or more pharmaceutically acceptable excipients.

12. Compound of formula (I) according to any one of Claims 1 to 5, in the form of a base, of a salt, of a hydrate or of a solvate which is pharmaceutically acceptable, for its use as a medicinal product.

13. Use of a compound of formula (I) according to any one of Claims 1 to 5, in the form of a base, of a salt, of a hydrate or of a solvate which is pharmaceutically acceptable, for preparing a medicinal product intended to prevent or treat a pathological condition in which endogenous cannabinoids and/or any other substrate metabolized by the FAAH enzyme are involved.

14. Use of a compound of formula (I) according to Claim 13, in the form of a base, of a salt, of a hydrate or of a solvate which is pharmaceutically acceptable, for preparing a medicinal product intended to prevent or treat acute or chronic pain, dizziness, vomiting, nausea, eating disorders, neurological and psychiatric pathological conditions, acute or chronic neurodegenerative diseases, epilepsy, sleep disorders, cardiovascular diseases, renal ischemia, cancers, disorders of the immune system, allergic diseases, parasitic, viral or bacterial infectious diseases, inflammatory diseases, osteoporosis, ocular conditions, pulmonary conditions, gastrointestinal diseases or urinary incontinence.

15. Use according to Claim 14, **characterized in that** the acute or chronic pain is of the neurogenic type, peripheral pain or pain associated with inflammatory diseases.

16. Use according to Claim 14, **characterized in that** the eating disorders correspond to anorexia and cachexia.

17. Use according to Claim 14, **characterized in that** the neurological and psychiatric conditions are chosen from the following conditions: shaking, dyskinesia, dystonia, spasticity, obsessive-compulsive behaviour, Tourette's syndrome, depression, anxiety, mood disorders and psychoses.

18. Use according to Claim 14, **characterized in that** the acute and chronic neurodegenerative diseases are chosen from the following diseases: Parkinson's disease, Alzheimer's disease, senile dementia, Huntington's chorea, lesions associated with cerebral ischemia and with cranial and medullary trauma.

19. Use according to Claim 14, **characterized in that** the cardiovascular diseases are chosen from the following diseases: hypertension, cardiac arythmias, arteriosclerosis, heart attack and cardiac ischemias.

20. Use according to Claim 14, **characterized in that** the cancers are chosen from benign skin tumours, papillomas, prostate tumours and brain tumours.

21. Use according to Claim 14, **characterized in that** the disorders of the immune system are autoimmune diseases.

22. Use according to Claim 14, **characterized in that** the allergic diseases are chosen from: immediate or delayed hypersensitivity, allergic rhinitis or conjunctivitis and contact dermatitis.

23. Use according to Claim 14, **characterized in that** the inflammatory diseases are diseases of the joints.

24. Use according to Claim 14, **characterized in that** the pulmonary conditions are chosen from: diseases of the respiratory tracts, bronchospasms, coughing, asthma, chronic bronchitis, chronic obstruction of the respiratory tracts and emphysema.

25. Use according to Claim 14, **characterized in that** the gastrointestinal diseases are chosen from: irritable bowel syndrome, intestinal inflammatory disorders, ulcers, diarrhoea and gastrooesophageal reflux.

## Patentansprüche

1. Verbindung der Formel (I) 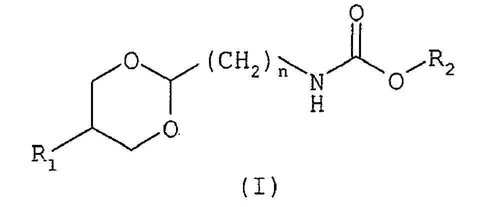 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy-, C₃₋₆-Cycloalkyl-O- oder C₃-₆-Cycloalkyl-C₁₋₃-alkoxygruppen substituierte Phenyl- oder Naphthalinylgruppe steht;
R₂ für eine Gruppe der allgemeinen Formel CHR₃CONHR₄, worin
R₃ für ein Wasserstoffatom oder eine Methylgruppe steht und
R₄ für ein Wasserstoffatom oder eine C₁₋₃-Alkyl-,
C₃₋₅-Cycloalkyl- oder (Pyridin-4-yl)methylgruppe steht,
oder eine 2,2,2-Trifluorethylgruppe
oder eine (Imidazol-2-yl)methylgruppe
oder eine (Benzmidazol-2-yl)methylgruppe
oder eine gegebenenfalls durch ein oder mehrere Halogenatome oder eine oder mehrere Cyano-, Nitro-, C₁₋₃-Alkyl-, C₁₋₃-Alkoxy-, Trifluormethyl- oder Trifluormethoxygruppen substituierte Phenylgruppe steht und
n für eine Zahl von 1 bis 3 steht;
in Basen-, Säureadditionssalz-, Hydrat- oder Solvatform.

2. Verbindung der Formel (I) nach Anspruch 1, **dadurch gekennzeichnet, daß** R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy-, C₃₋₆-Cycloalkyl-O- oder C₃₋₆-Cycloalkyl-C₁₋₃-alkoxygruppen substituierte Naphthalinylgruppe steht; in Basen-, Säureadditionssalz-, Hydrat- oder Solvatform.

3. Verbindung der Formel (I) nach Anspruch 1, **dadurch gekennzeichnet, daß** R₂ für eine Gruppe der allgemeinen Formel CHR₃CONHR₄, worin
R₃ für ein Wasserstoffatom steht und
R₄ für ein Wasserstoffatom oder eine C₁₋₃-Alkyl- oder (Pyridin-4-yl)methylgruppe steht,
oder eine 2,2,2-Trifluorethylgruppe
oder eine gegebenenfalls durch ein oder mehrere Halogenatome oder eine oder mehrere Cyano-, Nitro-, C₁₋₃-Alkyl-, C₁₋₃-Alkoxy-, Trifluormethyl- oder Trifluormethoxygruppen substituierte Phenylgruppe steht; in Basen-, Säureadditionssalz-, Hydrat- oder Solvatform.

4. Verbindung der Formel (I) nach Anspruch 1, **dadurch gekennzeichnet, daß** n für 2 oder 3 steht; in Basen-, Säureadditionssalz-, Hydrat- oder Solvatform.

5. Verbindung der Formel (I) nach einem der Ansprüche 1 bis 4, ausgewählt unter:
trans-3-[5-(Naphthalin-1-yl)-1,3-dioxan-2-yl]-propylcarbamidsäure-2-amino-2-oxoethylester;
*trans*-2-[5-(Naphthalin-1-yl)-1,3-dioxan-2-yl]-ethylcarbamidsäure-2-(methylamino)-2-oxoethylester;
*trans*-3-[5-(Naphthalin-1-yl)-1,3-dioxan-2-yl]-propylcarbamidsäure-2-(methylamino)-2-oxoethylester;
*trans*-2-[5-(Naphthalin-1-yl)-1,3-dioxan-2-yl]-ethylcarbamidsäure-2,2,2-trifluorethylester;
*trans*-3-[5-(Naphthalin-1-yl)-1,3-dioxan-2-yl]-propylcarbamidsäure-2,2,2-trifluorethylester;
*trans*-2-[5-(Naphthalin-1-yl)-1,3-dioxan-2-yl]-ethylcarbamidsäurephenylester;
*trans*-3-[5-(Naphthalin-1-yl)-1,3-dioxan-2-yl]-propylcarbamidsäurephenylester;
*trans*-3-[5-(4-Chlornaphthalin-1-yl)-1,3-dioxan-2-yl]propylcarbamidsäure-2-amino-2-oxoethylester;
*trans*-3-[5-(4-Chlornaphthalin-1-yl)-1,3-dioxan-2-yl]propylcarbamidsäure-2-(methylamino)-2-oxoethylester;
trans-3-[5-(6-Chlornaphthalin-1-yl)-1,3-dioxan-2-yl]propylcarbamidsäure-2-(methylamino)-2-oxoethylester;
*trans*-3-[5-(6-Methoxynaphthalin-1-yl)-1,3-dioxan-2-yl]propylcarbamidsäure-2-amino-2-oxoethylester;
cis-3-[5-(6-Methoxynaphthalin-1-yl)-1,3-dioxan-2-yl]propylcarbamidsäure-2-amino-2-oxoethylester;
*trans*-2-[5-(6-Methoxynaphthalin-1-yl)-1,3-dioxan-2-yl]ethylcarbamidsäure-2-(methylamino)-2-oxoethylester;
*trans*-2-[5-(6-Methoxynaphthalin-1-yl)-1,3-dioxan-2-yl]ethylcarbamidsäure-4-chlorphenylester;
*trans*-2-[5-(6-Methoxynaphthalin-1-yl)-1,3-dioxan-2-yl]ethylcarbamidsäure-2,2,2-trifluorethylester;
*trans*-3-[5-(6-Methoxynaphthalin-1-yl)-1,3-dioxan-2-yl]propylcarbamidsäure-2-(methylamino)-2-oxoethylester;
*trans*-3-[5-(6-Methoxynaphthalin-1-yl)-1,3-dioxan-2-yl]propylcarbamidsäure-2-(ethylamino)-2-oxoethylester;
*trans*-3-[5-(6-Methoxynaphthalin-1-yl)-1,3-dioxan-2-yl]propylcarbamidsäure-2-[(pyridin-4-yl)methylamino]-2-oxoethylester;
*trans*-3-[5-(6-Methoxynaphthalin-1-yl)-1,3-dioxan-2-yl]propylcarbamidsäure-2,2,2-trifluorethylester;
*trans*-3-[5-(6-Methoxynaphthalin-1-yl)-1,3-dioxan-2-yl]propylcarbamidsäurephenylester;
*trans*-3-[5-(6-Cyclopropylmethoxynaphthalin-1-yl)-1,3-dioxan-2-yl]propylcarbamidsäure-2-amino-2-oxoethylester;
*trans*-3-[5-(6-Cyclopropylmethoxynaphthalin-1-yl)-1,3-dioxan-2-yl]propylcarbamidsäure-4-chlorphenylester;
*trans*-3-[5-(6-Cyclopropylmethoxynaphthalin-1-yl)-1,3-dioxan-2-yl]propylcarbamidsäure-2,2,2-trifluorethylester;
*trans*-3-[5-(6-Phenylmethoxynaphthalin-1-yl)-1,3-dioxan-2-yl]propylcarbamidsäure-2-amino-2-oxoethylester;
*trans*-3-[5-(6-Hydroxynaphthalin-1-yl)-1,3-dioxan-2-yl]propylcarbamidsäure-2-amino-2-oxoethylester;
*trans*-3-[5-(6-Hydroxynaphthalin-1-yl)-1,3-dioxan-2-yl]propylcarbamidsäure-2-(methylamino)-2-oxoethylester;
*trans*-3-[5-(7-Methoxynaphthalin-1-yl)-1,3-dioxan-2-yl]propylcarbamidsäure-2-amino-2-oxoethylester;
*trans*-3-[5-(7-Methoxynaphthalin-1-yl)-1,3-dioxan-2-yl]propylcarbamidsäure-2-(methylamino)-2-oxoethylester;
*trans*-3-[5-(7-Methoxynaphthalin-1-yl)-1,3-dioxan-2-yl]propylcarbamidsäure-2,2,2-trifluorethylester;
*trans*-3-[5-(7-Methoxynaphthalin-1-yl)-1,3-dioxan-2-yl]propylcarbamidsäurephenylester;
*trans*-3-[5-(Naphthalin-2-yl)-1,3-dioxan-2-yl]-propylcarbamidsäurephenylester;
*trans*-3-[5-(Naphthalin-2-yl)-1,3-dioxan-2-yl]-propylcarbamidsäure-2-(methylamino)-2-oxoethylester;
trans-3-[5-(Naphthalin-2-yl)-1,3-dioxan-2-yl]-propylcarbamidsäure-2,2,2-trifluorethylester;
in Basen-, Säureadditionssalz-, Hydrat- oder Solvatform.

6. Verfahren zur Herstellung einer Verbindung der Formel (I) 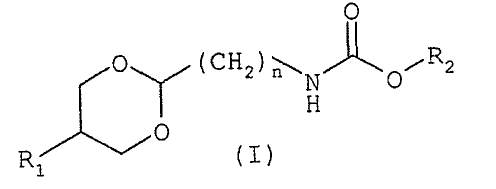 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy-, C₃₋₆-Cycloalkyl-O- oder C₃₋₆-Cycloalkyl-C₁₋₃-alkoxygruppen substituierte Phenyl- oder Naphthalinylgruppe steht;
R₂ für eine Gruppe der allgemeinen Formel CHR₃CONHR₄, worin R₃ für ein Wasserstoffatom oder eine Methylgruppe steht und R₄ für ein Wasserstoffatom oder eine C₁₋₃-Alkyl-, C₃₋₅-Cycloalkyl- oder (Pyridin-4-yl)methylgruppe steht, steht und
n für eine Zahl von 1 bis 3 steht;
bei dem man
den Carbamatester der allgemeinen Formel (Ia) 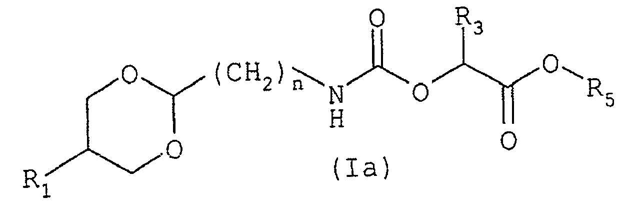 worin R₁, R₃ und n die in der allgemeinen Formel (I) angegebene Bedeutung besitzen und R₅ für eine Methyl- oder Ethylgruppe steht,
entweder durch direkte Aminolyse mit einem Amin der allgemeinen Formel R₄NH₂, worin R₄ die in der allgemeinen Formel (I) angegebene Bedeutung besitzt, oder durch Hydrolyse zu einer Säure der Formel (Ia), worin R₅ für ein Wasserstoffatom steht, und nachfolgende Kupplung mit einem Amin der allgemeinen Formel R₄NH₂, worin R₄ die in der allgemeinen Formel (I) angegebene Bedeutung besitzt, in eine Verbindung der allgemeinen Formel (I) umwandelt.

7. Verfahren zur Herstellung einer Verbindung der Formel (I) 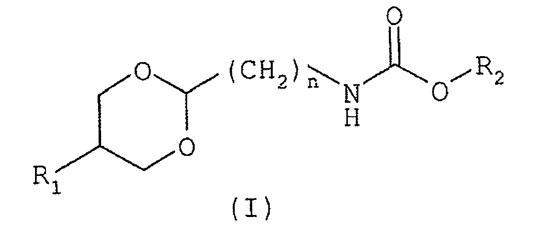 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy-, C₃₋₆-Cycloalkyl-O- oder C₃₋₆-Cycloalkyl-C₁₋₃-alkoxygruppen substituierte Phenyl- oder Naphthalinylgruppe steht;
R₂ für eine Gruppe der allgemeinen Formel CHR₃CONHR₄, worin R₃ für ein Wasserstoffatom oder eine Methylgruppe steht und R₄ für ein Wasserstoffatom oder eine C₁₋₃-Alkyl-, C₃₋₅-Cycloalkyl- oder (Pyridin-4-yl)methylgruppe steht, steht und
n für eine Zahl von 1 bis 3 steht;
bei dem man
das Oxazolidindionderivat der allgemeinen Formel (VI) 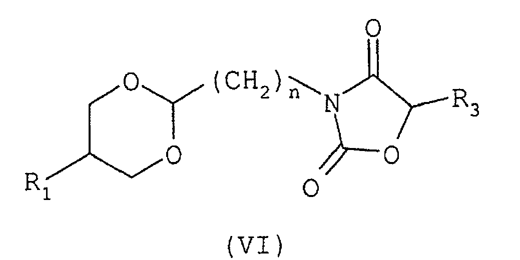 worin R₁, R₃ und n die in der allgemeinen Formel (I) angegebene Bedeutung besitzen,
durch Aminolyse mit einem Amin der allgemeinen Formel R₄NH₂, worin R₄ die in der allgemeinen Formel (I) angegebene Bedeutung besitzt, in eine Verbindung der allgemeinen Formel (I) umwandelt.

8. Verfahren zur Herstellung einer Verbindung der Formel (I) 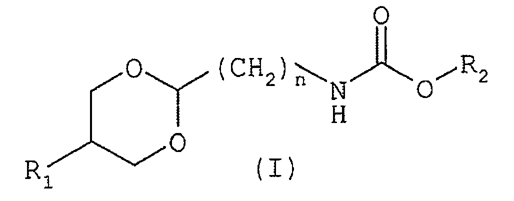 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy-, C₃₋₆-Cycloalkyl-O- oder C₃₋₆-Cycloalkyl-C₁₋₃-alkoxygruppen substituierte Phenyl- oder Naphthalinylgruppe steht;
R₂ für eine Gruppe der allgemeinen Formel CHR₃CONHR₄, worin
R₃ für ein Wasserstoffatom oder eine Methylgruppe steht und
R₄ für ein Wasserstoffatom oder eine C₁₋₃-Alkyl-,
C₃₋₅-Cycloalkyl- oder (Pyridin-4-yl)methylgruppe steht,
oder eine 2,2,2-Trifluorethylgruppe
oder eine (Imidazol-2-yl)methylgruppe
oder eine (Benzmidazol-2-yl)methylgruppe
oder eine gegebenenfalls durch ein oder mehrere Halogenatome oder eine oder mehrere Cyano-, Nitro-, C₁₋₃-Alkyl-, C₁₋₃-Alkoxy-, Trifluormethyl- oder Trifluormethoxygruppen substituierte Phenylgruppe steht und
n für eine Zahl von 1 bis 3 steht;
bei dem man
ein Amin der allgemeinen Formel (II) 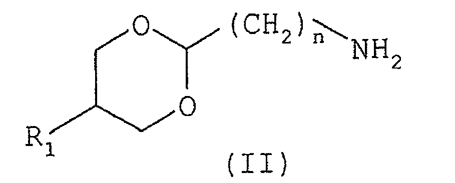 worin R₁ und n die in der allgemeinen Formel (I) angegebene Bedeutung besitzen, mit einem Kohlensäureester der allgemeinen Formel (III) 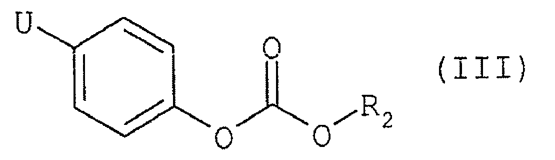 worin U für ein Wasserstoffatom oder eine Nitrogruppe steht und R₂ die in der allgemeinen Formel (I) angegebene Bedeutung besitzt, umsetzt.

9. Verbindung der allgemeinen Formel (Ia) 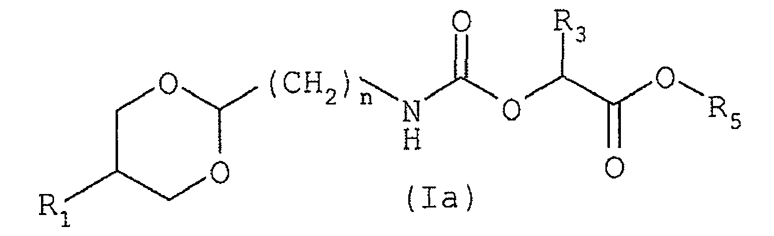 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy- oder C₃₋₆-Cycloalkyl-C₁₋₃-alkoxygruppen substituierte Phenyl- oder Naphthalinylgruppe steht;
R₃ für ein Wasserstoffatom oder eine Methylgruppe steht;
R₅ für ein Wasserstoffatom oder eine Methyl- oder Ethylgruppe steht und
n für eine Zahl von 1 bis 3 steht.

10. Verbindung der allgemeinen Formel (VI) 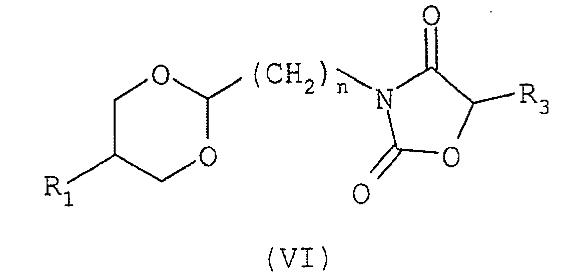 worin
R₁ für eine gegebenenfalls durch ein oder mehrere Halogenatome oder eine oder mehrere Hydroxy-, Cyano-, Nitro-, C₁₋₃-Alkyl-, C₁₋₃-Alkoxy-, Trifluormethyl-, Trifluormethoxy-, Benzyloxy- oder C₃₋₆-Cycloalkyl-C₁₋₃-alkoxygruppen substituierte Phenyl- oder Naphthalinylgruppe steht;
R₃ für ein Wasserstoffatom oder eine Methylgruppe steht und
n für eine Zahl von 1 bis 3 steht.

11. Pharmazeutische Zusammensetzung, enthaltend mindestens eine Verbindung der Formel (I) nach einem der Ansprüche 1 bis 5 in pharmazeutisch unbedenklicher Basen-, Salz-, Hydrat- oder Solvatform und gegebenenfalls einen oder mehrere pharmazeutisch unbedenkliche Trägerstoffe.

12. Verbindung der Formel (I) nach einem der Ansprüche 1 bis 5 in pharmazeutisch unbedenklicher Basen-, Salz-, Hydrat- oder Solvatform zur Verwendung als Arzneimittel.

13. Verwendung einer Verbindung der Formel (I) nach einem der Ansprüche 1 bis 5 in pharmazeutisch unbedenklicher Basen-, Salz-, Hydrat- oder Solvatform zur Herstellung eines Arzneimittels zur Prävention oder Behandlung einer Pathologie, an der endogene Cannabinoide und/oder andere durch das Enzym FAAH metabolisierte Substanzen beteiligt sind.

14. Verwendung einer Verbindung der Formel (I) nach Anspruch 13 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebserkrankungen, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.

15. Verwendung nach Anspruch 14, **dadurch gekennzeichnet, daß** die akuten oder chronischen Schmerzen vom neurogenen Typ, peripher oder mit entzündlichen Erkrankungen assoziiert sind.

16. Verwendung nach Anspruch 14, **dadurch gekennzeichnet, daß** die Eßstörungen Anorexien und Kachexien entsprechen.

17. Verwendung nach Anspruch 14, **dadurch gekennzeichnet, daß** die neurologischen und psychiatrischen Pathologien unter den folgenden Pathologien ausgewählt sind: Zittern, Dyskinesien, Dystonien, Spastizität, Zwangsneurosen, Tourette-Syndrom, Depression, Angst, Gemütsstörungen, Psychosen.

18. Verwendung nach Anspruch 14, **dadurch gekennzeichnet, daß** die akuten und chronischen neurodegenerativen Erkrankungen unter den folgenden Erkrankungen ausgewählt sind: Parkinson-Krankheit, Alzheimer-Krankheit, senile Demenz, Chorea Huntington, Läsionen in Verbindung mit Hirnischämie und Schädel- und Knochenmarkstraumata.

19. Verwendung nach Anspruch 14, **dadurch gekennzeichnet, daß** die Herz-Kreislauf-Erkrankungen unter den folgenden Erkrankungen ausgewählt sind: Hypertonie, Herzarrhythmien, Arteriosklerose, Herzanfall, Herzischämien.

20. Verwendung nach Anspruch 14, **dadurch gekennzeichnet, daß** die Krebserkrankungen unter gutartigen Hauttumoren, Papillomen, Prostatatumoren und Hirntumoren ausgewählt sind.

21. Verwendung nach Anspruch 14, **dadurch gekennzeichnet, daß** es sich bei den Störungen des Immunsystems um Autoimmunerkrankungen handelt.

22. Verwendung nach Anspruch 14, **dadurch gekennzeichnet, daß** die allergischen Erkrankungen ausgewählt sind unter: Überempfindlichkeitsreaktion vom Sofort- oder Spät-Typ, allergischer Rhinitis oder Konjunktivitis, Kontaktdermatitis.

23. Verwendung nach Anspruch 14, **dadurch gekennzeichnet, daß** es sich bei den entzündlichen Erkrankungen um Gelenkserkrankungen handelt.

24. Verwendung nach Anspruch 14, **dadurch gekennzeichnet, daß** es sich bei den Lungenleiden um Erkrankungen der Atemwege, Bronchospasmen, Husten, Asthma, chronische Bronchitis, chronischobstruktive Atemwegserkrankung oder Emphysem handelt.

25. Verwendung nach Anspruch 14, **dadurch gekennzeichnet, daß** die Magen-Darm-Erkrankungen ausgewählt sind unter: Reizkolon, entzündlichen Darmerkrankungen, Geschwüren, Diarrhöen, gastroösophagealem Reflux.
